# EUROPEAN PATENT SPECIFICATION

(11) **EP 1 651 189 B1**
(45) Date of publication and mention of the grant of the patent: **03.12.2008**
(21) Application number: 04776615.9
(22) Date of filing: 26.07.2004
(51) Int. Cl.: A61K 9/14

(54) **NOVEL METAXALONE COMPOSITIONS**
NEUE METAXALON-ZUSAMMENSETZUNGEN
NOUVELLES COMPOSITIONS DE METAXALONE

(30) Priority: 08.08.2003 US 493446 P
(43) Date of publication of application: 03.05.2006
(73) Proprietor: Elan Pharma International Limited, Athlone County Westmeath (IE)
(72) Inventor: PRUITT, John, D., Suwanee, GA 30024-4062 (US); RYDE, Tuula, A., Malvern, PA 19355 (US); BOSCH, William, H., Bryn Mawr, PA 19010 (US)
(74) Representative: Ryan, Anne Mary
(86) International application number: PCT/US2004/019108
(87) International publication number: WO 2005/016310

(56) References cited:
- EP-A- 0 275 796
- WO-A1-03/037379
- WO-A1-20/04019937
- WO-A2-20/04105694
- US-A- 5 510 118

## Description

### FIELD OF THE INVENTION

The present invention relates to novel compositions of metaxalone, comprising metaxalone particles having an effective average particle size of less than about 2000 nm and at least one surface stabilizer that is adsorbed on the surface of the drug particles.

### BACKGROUND OF THE INVENTION

### A. Background Regarding Nanoparticulate Compositions

Nanoparticulate compositions, first described in U.S. Patent No. 5,145,684 ("the '684 patent"), are particles consisting of a poorly soluble therapeutic or diagnostic agent having associated with the surface thereof a non-crosslinked surface stabilizer. The '684 patent does not describe nanoparticulate compositions metaxalone.

Methods of making nanoparticulate compositions are described, for example, in U.S. Patent Nos. 5,518,187 and 5,862,999, both for "Method of Grinding Pharmaceutical Substances;" U.S. Patent No. 5,718,388, for "Continuous Method of Grinding Pharmaceutical Substances;" and U.S. Patent No. 5,510,118 for "Process of Preparing Therapeutic Compositions Containing Nanoparticles." These patents do not describe methods of making nanoparticulate metaxalone.

Nanoparticulate compositions are also described, for example, in U.S. Patent Nos. 5,298,262 for "use of Ionic Cloud Point Modifiers to Prevent Particle Aggregation During Sterilization;" 5,302,401 for "Method to Reduce Particle Size Growth During Lyophilization;" 5,318,767 for "X-Ray Contrast Compositions Useful in Medical Imaging;" 5,326,552 for "Novel Formulation For Nanoparticulate X-Ray Blood Pool Contrast Agents Using High Molecular Weight Non-ionic Surfactants;" 5,328,404 for "Method of X-Ray Imaging Using Iodinated Aromatic Propanedioates;" 5,336,507 for "Use of Charge Phospholipids to Reduce Nanoparticle Aggregation;" 5,340,564 for "Formulations Comprising Olin 10-G to Prevent Particle Aggregation and Increase Stability;" 5,346,702 for "Use of Non-Ionic Cloud Point Modifiers to Minimize Nanoparticulate Aggregation During Sterilization;" 5,349,957 for "Preparation and Magnetic Properties of Very Small Magnetic-Dextran Particles;" 5,352,459 for "Use of Purified Surface Modifiers to Prevent Particle Aggregation During Sterilization;" 5,399,363 and 5,494,683, both for "Surface Modified Anticancer Nanoparticles;" 5,401,492 for "Water Insoluble Non-Magnetic Manganese Particles as Magnetic Resonance Enhancement Agents;" 5,429,824 for "Use of Tyloxapol as a Nanoparticulate Stabilizer;" 5,447,710 for "Method for Making Nanoparticulate X-Ray Blood Pool Contrast Agents Using High Molecular Weight Non-ionic Surfactants;" 5,451,393 for "X-Ray Contrast Compositions Useful in Medical Imaging;" 5,466,440 for "Formulations of Oral Gastrointestinal Diagnostic X-Ray Contrast Agents in Combination with Pharmaceutically Acceptable Clays;" 5,470,583 for "Method of Preparing Nanoparticle Compositions Containing Charged Phospholipids to Reduce Aggregation;" 5,472,683 for "Nanoparticulate Diagnostic Mixed Carbamic Anhydrides as X-Ray Contrast Agents for Blood Pool and Lymphatic System Imaging;" 5,500,204 for "Nanoparticulate Diagnostic Dimers as X-Ray Contrast Agents for Blood Pool and Lymphatic System Imaging;" 5,518,738 for "Nanoparticulate NSAID Formulations;" 5,521,218 for "Nanoparticulate Iododipamide Derivatives for Use as X-Ray Contrast Agents;" 5,525,328 for "Nanoparticulate Diagnostic Diatrizoxy Ester X-Ray Contrast Agents for Blood Pool and Lymphatic System Imaging;" 5,543,133 for "Process of Preparing X-Ray Contrast Compositions Containing Nanoparticles;" 5,552,160 for "Surface Modified NSAID Nanoparticles;" 5,560,931 for "Formulations of Compounds as Nanoparticulate Dispersions in Digestible Oils or Fatty Acids;" 5,565,188 for "Polyalkylene Block Copolymers as Surface Modifiers for Nanoparticles;" 5,569,448 for "Sulfated Non-ionic Block Copolymer Surfactant as Stabilizer Coatings for Nanoparticle Compositions;" 5,571,536 for "Formulations of Compounds as Nanoparticulate Dispersions in Digestible Oils or Fatty Acids;" 5,573,749 for "Nanoparticulate Diagnostic Mixed Carboxylic Anydrides as X-Ray Contrast Agents for Blood Pool and Lymphatic System Imaging;" 5,573,750 for "Diagnostic Imaging X-Ray Contrast Agents;" 5,573,783 for "Redispersible Nanoparticulate Film Matrices With Protective Overcoats;" 5,580,579 for "Site-specific Adhesion Within the GI Tract Using Nanoparticles Stabilized by High Molecular Weight, Linear Poly(ethylene Oxide) Polymers;" 5,585,108 for "Formulations of Oral Gastrointestinal Therapeutic Agents in Combination with Pharmaceutically Acceptable Clays;" 5,587,143 for "Butylene Oxide-Ethylene Oxide Block Copolymers Surfactants as Stabilizer Coatings for Nanoparticulate Compositions;" 5,591,456 for "Milled Naproxen with Hydroxypropyl Cellulose as Dispersion Stabilizer;" 5,593,657 for "Novel Barium Salt Formulations Stabilized by Non-ionic and Anionic Stabilizers;" 5,622,938 for "Sugar Based Surfactant for Nanocrystals;" 5,628,981 for "Improved Formulations of Oral Gastrointestinal Diagnostic X-Ray Contrast Agents and Oral Gastrointestinal Therapeutic Agents;" 5,643,552 for "Nanoparticulate Diagnostic Mixed Carbonic Anhydrides as X-Ray Contrast Agents for Blood Pool and Lymphatic System Imaging;" 5,718,388 for "Continuous Method of Grinding Pharmaceutical Substances;" 5,718,919 for "Nanoparticles Containing the R(-)Enantiomer of Ibuprofen;" 5,747,001 for "Aerosols Containing Beclomethasone Nanoparticle Dispersions;" 5,834,025 for "Reduction of Intravenously Administered Nanoparticulate Formulation Induced Adverse Physiological Reactions;" 6,045,829 "Nanocrystalline Formulations of Human Immunodeficiency Virus (HIV) Protease Inhibitors Using Cellulosic Surface Stabilizers;" 6,068,858 for "Methods of Making Nanocrystalline Formulations of Human Immunodeficiency Virus (HIV) Protease Inhibitors Using Cellulosic Surface Stabilizers;" 6,153,225 for "Injectable Formulations of Nanoparticulate Naproxen;" 6,165,506 for "New Solid Dose Form of Nanoparticulate Naproxen;" 6,221,400 for "Methods of Treating Mammals Using Nanocrystalline Formulations of Human Immunodeficiency Virus (HIV) Protease Inhibitors;" 6,264,922 for "Nebulized Aerosols Containing Nanoparticle Dispersions;" 6,267,989 for "Methods for Preventing Crystal Growth and Particle Aggregation in Nanoparticle Compositions;" 6,270,806 for "Use of PEG-Derivatized Lipids as Surface Stabilizers for Nanoparticulate Compositions;" 6,316,029 for "Rapidly Disintegrating Solid Oral Dosage Form," 6,375,986 for "Solid Dose Nanoparticulate Compositions Comprising a Synergistic Combination of a Polymeric Surface Stabilizer and Dioctyl Sodium Sulfosuccinate," 6,428,814 for "Bioadhesive nanoparticulate compositions having cationic surface stabilizers;" 6,431,478 for "Small Scale Mill;" 6,432,381 for "Methods for Targeting Drug Delivery to the Upper and/or Lower Gastrointestinal Tract," 6,592,903 for "Nanoparticulate Dispersions Comprising a Synergistic Combination of a Polymeric Surface Stabilizer and Dioctyl Sodium Sulfosuccinate," 6,582,285 for "Apparatus for sanitary wet milling;" 6,656,504 for "Nanoparticulate Compositions Comprising Amorphous Cyclosporine;" 6,742,734 for "System and Method for Milling Materials;" and 6,745,962 for "Small Scale Mill and Method Thereof;" In addition, U.S. Patent Application No. 20020012675 A1, published on January 31, 2002, for "Controlled Release Nanoparticulate Compositions," and WO 02/098565 for "System and Method for Milling Materials," describe nanoparticulate active agent compositions. None of these references describe nanoparticulate compositions of metaxalone.

Amorphous small particle compositions are described, for example, in U.S. Patent Nos. 4,783,484 for "Particulate Composition and Use Thereof as Antimicrobial Agent;" 4,826,689 for "Method for Making Uniformly Sized Particles from Water-Insoluble Organic Compounds;" 4,997,454 for "Method for Making Uniformly-Sized Particles From Insoluble Compounds;" 5,741,522 for "Ultrasmall, Non-aggregated Porous Particles of Uniform Size for Entrapping Gas Bubbles Within and Methods;" and 5,776,496, for "Ultrasmall Porous Particles for Enhancing Ultrasound Back Scatter." These references do not describe nanoparticulate metaxalone.

### B. Background Regarding Metaxalone

Metaxalone is a skeletal muscle relaxant used to relieve the pain of muscle injuries, spasms, sprains, and strains. The mechanism of action of metaxalone in humans has not been established, but may be due to general central nervous system depression. It has no direct action on the contractile mechanism of striated muscle, the motor end plate, or the nerve fiber. The drug does not directly relax tense skeletal muscles in man. *See* The Physician's Desk Reference, 57th Edition, p. 1274 (Thompson PDR, Montvale NJ, 2003). Metaxalone is indicated as an adjunct to rest, physical therapy, and other measures for the relief of discomforts associated with acute, painful musculoskeletal conditions.

Metaxalone is a tasteless, odorless, white crystalline powder that melts without decomposition at 121.5 to 123 °C. The compound has the chemical name 5-[(3,4-dimethylphenoxy)methyl]-2-oxazolidinone, and the following chemical structure: 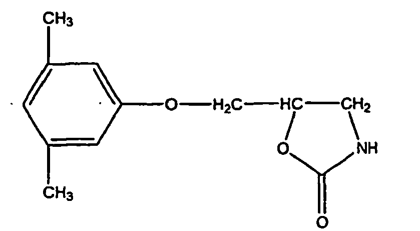 The chemical structure of metaxalone is unrelated to that of other skeletal muscle relaxants. *See* http://www.aanos.org/edctn_msk_disordr.htm.

Metaxalone is contraindicated in individuals who have shown hypersensitivity to the drug. Metaxalone is also contraindicated for patients with a known tendency to drug induced, hemolytic, or other anemias. It is contraindicated in patients with significantly impaired renal or hepatic function.

The most frequent reactions to metaxalone include nausea, vomiting, gastrointestinal upset, drowsiness, dizziness, headache, and nervousness or "irritability." Other adverse reactions include hypersensitivity reaction, characterized by a light rash with or without pruritus, leukopenia, hemolytic anemia, and jaundice.

Metaxalone is marketed under the brand name SKELAXIN^{®} (Elan Pharmaceuticals, Inc.) in 400 mg and 800 mg tablets. The general dosage for adults and children over 12 years of age is two 400 mg tablets (800 mg) or one 800 mg tablet, three to four times a day.

The pharmacokinetics of SKELAXIN^{®} are provided in New Drug Application No. 13-217/S-036; see http://www.fda.gov/cder/foi/label/2002/13217s0361b1.pdf. Specifically, in a single center randomized, two-period crossover study in 42 healthy volunteers (31 males, 11 females), a single 400 mg SKELAXIN^{®} (metaxalone) tablet was administered under both fasted and fed conditions. Under fasted conditions, mean peak plasma concentrations (Cₘₐₓ) of 865.3 ng/mL were achieved within 3.3 +/- 1.2 hours (S.D.) after dosing (Tₘₐₓ). Metaxalone concentrations declined with a mean terminal half-life (t 1/2) of 9.2 +/- 4.8 hours. The mean apparent oral clearance (CL/F) of metaxalone was 68 +/- 34 L/h.

In the same study, following a standardized high fat meal, food statistically significantly increased the rate (Cₘₐₓ) and extent of absorption (AUC₍₀₋ₜ₎, AUCinf) of metaxalone from SKELAXIN^{®} tablets. Relative to the fasted treatment the observed increases were 177.5%, 123.5%, and 115.4%, respectively. The mean Tₘₐₓ was also increased to 4.3 +/- 2.3 hours, whereas the mean t 1/2 was decreased to 2.4 +/- 1.2 hours. This decrease in half-life over that seen in the fasted subjects is felt to be due to the more complete absorption of metaxalone in the presence of a meal resulting in a better estimate of half-life. The mean apparent oral clearance (CL/F) of metaxalone was relatively unchanged relative to fasted administration (59 +/- 29 L/hr). Although a higher Cₘₐₓ and AUC were observed after the administration of SKELAXIN^{®} (metaxalone) with a standardized high fat meal, the clinical relevance of these effects is unknown.

In another single center, randomized four-period crossover study in 59 healthy volunteers (37 males, 22 females), the rate and extent of metaxalone absorption were determined after the administration of SKELAXIN^{®} tablets under both fasted and fed conditions. Under fasted conditions, the administration of two SKELAXIN^{®} 400 mg tablets produced peak plasma metaxalone concentrations (Cₘₐₓ) of 1653 ng/mL 3.0 + 1.2 hours after dosing (Tₘₐₓ). Metaxalone concentrations declined with mean terminal half-life (t½) of 8.0 + 4.6 hours. The mean apparent oral clearance (CL/F) of metaxalone was 66 + 34 L/hr. Except for a 17% decrease in mean Cₘₐₓ, these values were not statistically different from those after the administration of one SKELAXIN^{®} 800 mg tablet.

In the same study, the administration of two SKELAXIN^{®} 400 mg tablets following a standardized high fat meal showed an increase in the mean Cₘₐₓ, and the area under the curve (AUC_{0-inf}) of metaxalone by 194% and 142%, respectively. A high fat meal also increased the mean Tₘₐₓ to 4.9 +/- 2.3 hours but decreased the mean t ½ to 4.2 + 2.5 hr. The effect of a high fat meal on the absorption of metaxalone from one SKELAXIN^{®} 800 mg tablet was very similar to that on the absorption from two SKELAXIN^{®} 400 mg tablets in quality and quantity. The clinical relevance of these effects is unknown.

The absolute bioavailability of metaxalone from SKELAXIN^{®} tablets is not known. Metaxalone is metabolized by the liver and excreted in the urine as unidentified metabolites. The impact of age, gender, hepatic, and renal disease on the pharmacokinetics of SKELAXIN^{®} (metaxalone) has not been determined.

Other prior descriptions of metaxalone include U.S. Patent No. 6,407,128 for "Method for Increasing the Bioavailability of Metaxalone," which describes a method of administering metaxalone in combination with food.

U.S. Patent No. 6,572,880 for "Methods and Transdermal Compositions for Pain Relief" describes compositions of an amine containing compound having biphasic solubility and an agent which enhances the activity of the amine containing compound, such as metaxalone. The compositions are described as being useful in relieving pain.

U.S. Patent No. 6,592,980 for "Method for Producing 5-aryloxymethyl-2-oxazolidinones" teaches a method for making the subject compounds comprising fusing a triglycidyl isocyanurate (TGIC) with an unsubstituted or a mono- or di-sobstituted phenol. In addition, U.S. Patent No. 6,538,142 for "Process for the Preparation of Metaxalone" describes a reaction/reduction process for obtaining the desired compound.

U.S. Patent No. 4,722,938, for "Methods for Using Musculoskeletal Relaxants," describes methods of using musculoskeletal relaxants such as metaxalone.

Finally, U.S. Patent No. 6,562,363 for "Bioadhesive Compositions and Methods for Topical Administration of Active Agents," describes bioadhesive compositions for topical application to skin or mucous membranes. The compositions comprise an admixture of: (1) at least one PVP polymer; (2) at least one bioadhesive solvent suitable for use with an active agent; and (3) an active agent such as metaxalone.

WO 2004/105694, published after the priority date of the present application, discloses pharmaceutical compositions and dosage forms for administration of hydrophobic drugs. The pharmaceutical compositions include a hydrophobic drug, preferably a steroid; a solubilizer, preferably a vitamin E substance; and a surfactant. A synergistic effect between the hydrophobic drug and the vitamin E substance results in improved dispersion of the active agent and the solubilizer.

There is a need in the art for metaxalone compositions which can decrease frequency of dosing, improve clinical efficacy, and potentially reduce side effects. The present invention satisfies these needs.

### SUMMARY OF THE INVENTION

The present invention relates to nanoparticulate compositions comprising metaxalone. The compositions comprise metaxalone and at least one surface stabilizer adsorbed on the surface of the metaxalone particles. The nanoparticulate metaxalone particles have an effective average particle size of less than about 2 microns.

Another aspect of the invention relates to the use of such a composition for the preparation of a medicament.

We further describe to pharmaceutical compositions comprising such a nanoparticulate metaxalone composition. The pharmaceutical compositions preferably comprise metaxalone, at least one surface stabilizer, and at least one pharmaceutically acceptable carrier, as well as any desired excipients.

Advantages and properties of the compositions of the invention are described herein.

The invention further discloses a method of making a nanoparticulate metaxalone composition. Such a method comprises contacting metaxalone and at least one surface stabilizer for a time and under conditions sufficient to provide a nanoparticulate metaxalone composition in which the surface stabilizer is adsorbed on the surface of the metaxalone particles. The one or more surface stabilizers can be contacted with metaxalone either before, preferably during, or after size reduction of the metaxalone.

We also describe methods of treatment using the nanoparticulate metaxalone compositions of the invention for treatment of musculoskeletal disorders.

### BRIEF DESCRIPTION OF THE FIGURES

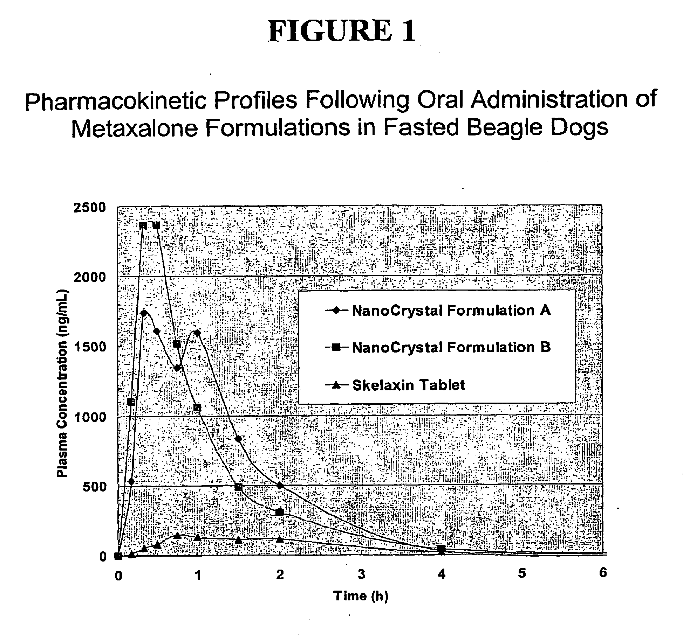

- FIGURE 1:: Graphically shows the average concentration (ng/mL) of metaxalone over a six hour time period, under fasted conditions, following oral administration to four male dogs of a 100 mg metaxalone dosage of: (1) nanoparticulate metaxalone dispersion #1; (2) nanoparticulate metaxalone dispersion #2; and (3) ¼ of a SKELAXIN^{®} tablet; and

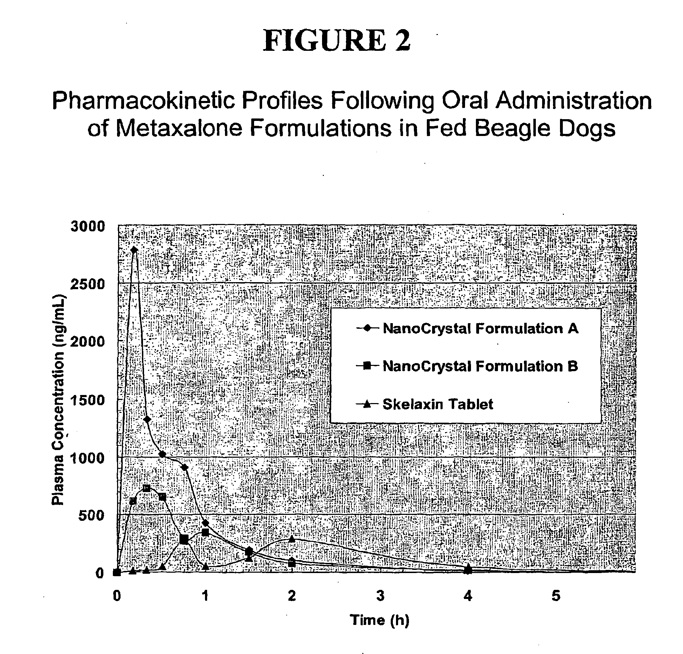

- FIGURE 2:: Graphically shows the average concentration (ng/mL) of metaxalone over a six hour time period, under fed conditions, following oral administration to four male dogs of a 100 mg metaxalone dosage of: (1) nanoparticulate metaxalone dispersion #1; (2) nanoparticulate metaxalone dispersion #2; and (3) ¼ of a SKELAXIN^{®} tablet.

### DETAILED DESCRIPTION

The present invention is directed to nanoparticulate compositions comprising metaxalone according to claim 1. The compositions comprise metaxalone and at least one surface stabilizer that is adsorbed on the surface of the drug. The nanoparticulate metaxalone particles have an effective average particle size of less than 2 microns.

As taught in the '684 patent, not every combination of surface stabilizer and active agent will result in a stable nanoparticulate composition. It was surprisingly discovered that stable nanoparticulate metaxalone formulations can be made.

The current formulations of metaxalone suffer from the following problems: (1) the poor solubility of the drug results in a relatively low bioavailability; (2) dosing must be repeated several times each day; and (3) a wide variety of side effects are associated with the current dosage forms of the drug.

The present invention overcomes problems encountered with the prior art metaxalone formulations. Specifically, the nanoparticulate metaxalone formulations of the invention may offer the following advantages: (1) faster onset of action; (2) a potential decrease in the frequency of dosing; (3) smaller doses of metaxalone required to obtain the same pharmacological effect; (4) increased bioavailability; (5) an increased rate of dissolution; (6) improved performance characteristics for oral, intravenous, subcutaneous, or intramuscular injection, such as higher does loading and smaller tablet or liquid dose volumes; (7) improved pharmacokinetic profiles, such as improved Tₘₐₓ, Cₘₐₓ, and AUC profiles; (8) substantially similar or bioequivalent pharmacokinetic profiles of the nanoparticulate metaxalone compositions when administered in the fed versus the fasted state; (9) bioadhesive metaxalone formulations, which can coat the gut or the desired site of application and be retained for a period of time, thereby increasing the efficacy of the drug as well as eliminating or decreasing the frequency of dosing; (10) high redispersibility of the nanoparticulate metaxalone particles present in the compositions of the invention following administration; (11) the nanoparticulate metaxalone compositions can be formulated in a dried form which readily redisperses; (12) low viscosity liquid nanoparticulate metaxalone dosage forms can be made; (13) for liquid nanoparticulate metaxalone compositions having a low viscosity - better subject compliance due to the perception of a lighter formulation which is easier to consume and digest; (14) for liquid nanoparticulate metaxalone compositions having a low viscosity - ease of dispensing because one can use a cup or a syringe; (15) the nanoparticulate metaxalone compositions can be used in conjunction with other active agents; (16) the nanoparticulate metaxalone compositions can be sterile filtered; (17) the nanoparticulate metaxalone compositions are suitable for parenteral administration; and (18) the nanoparticulate metaxalone compositions do not require organic solvents or pH extremes.

A preferred dosage form of the invention is a solid dosage form, although any pharmaceutically acceptable dosage form can be utilized. Exemplary solid dosage forms include, but are not limited to, tablets, capsules, sachets, lozenges, powders, pills, or granules. The solid dosage form can be, for example, a fast melt dosage form, controlled release dosage form, lyophilized dosage form, delayed release dosage form, extended release dosage form, pulsatile release dosage form, mixed immediate release and controlled release dosage form, or a combination thereof. A solid dose tablet formulation is preferred.

The present invention is described herein using several definitions, as set forth below and throughout the application.

As used herein, "about" will be understood by persons of ordinary skill in the art and will vary to some extent on the context in which it is used. If there are uses of the term which are not clear to persons of ordinary skill in the art given the context in which it is used, "about" will mean up to plus or minus 10% of the particular term.

"Conventional" or "non-nanoparticulate active agent" shall mean an active agent which is solubilized or which has an effective average particle size of greater than about 2 microns. Nanoparticulate active agents as defined herein have an effective average particle size of less than about 2 microns.

"Poorly water soluble drugs" as used herein means those having a solubility of less than about 30 mg/ml, preferably less than about 20 mg/ml, preferably less than about 10 mg/ml, or preferably less than about 1 mg/ml. Such drugs tend to be eliminated from the gastrointestinal tract before being absorbed into the circulation.

As used herein with reference to stable drug particles, 'stable' includes, but is not limited to, one or more of the following parameters: (1) that the metaxalone particles do not appreciably flocculate or agglomerate due to interparticle attractive forces, or otherwise significantly increase in particle size over time; (2) that the physical structure of the metaxalone particles is not altered over time, such as by conversion from an amorphous phase to crystalline phase; (3) that the metaxalone particles are chemically stable; and/or (4) where the metaxalone has not been subject to a heating step at or above the melting point of the metaxalone in the preparation of the nanoparticles of the invention.

'Therapeutically effective amount' as used herein with respect to a drug dosage, shall mean that dosage that provides the specific pharmacological response for which the drug is administered in a significant number of subjects in need of such treatment. It is emphasized that 'therapeutically effective amount,' administered to a particular subject in a particular instance will not always be effective in treating the diseases described herein, even though such dosage is deemed a 'therapeutically effective amount' by those skilled in the art. It is to be further understood that drug dosages are, in particular instances, measured as oral dosages, or with reference to drug levels as measured in blood.

### A. Preferred Characteristics of the Nanoparticulate Metaxalone Compositions of the Invention

### 1. Fast Onset of Activity

The use of conventional formulations of metaxalone is not ideal due to delayed onset of action. In contrast, the nanoparticulate metaxalone compositions of the invention exhibit faster therapeutic effects.

When the nanoparticulate metaxalone compositions of the invention are formulated into an oral dosage form (*e*.*g*., the dosage form of SKELAXIN^{®}), peak plasma concentration of the nanoparticulate metaxalone can be obtained (Tₘₐₓ) in less than about 2 hours. In other embodiments of the invention, peak plasma concentration of the nanoparticulate metaxalone can be obtained in less than about 110 min., less than about 100 min., less than about 90 min., less than about 80 min. less than about 70 min., less than about 60 min., less than about 50 min., less than about 40 min., less than about 30 min., less than about 25 min., less than about 20 min., less than about 15 min., or less than about 10 min.

As shown in the examples below, exemplary nanoparticulate metaxalone compositions exhibited a Tₘₐₓ of 0.38 hr and 0.40 hr under fed conditions, and 0.37 hr and 0.60 hr under fasted conditions. This was in contrast to the Tₘₐₓ of SKELAXIN^{®} OF 2.19 hrs and 1.19 hrs under fed and fasted conditions, respectively.

### 2. Frequency of Dosing and Dosage Quantity

The recommended total daily dose of SKELAXIN^{®} is 800 mg administered 3-4 times daily, for a total daily dose of 2400 to 3200 mg/day. *See* Physicians' Desk Reference, 57th Edition, pp. 1274 (2003).

In contrast, the metaxalone compositions of the invention may be administered less frequently and at lower doses in dosage forms such as liquid dispersions, powders, sprays, solid re-dispersable dosage forms, ointments, creams, *etc.* Exemplary types of formulations useful in the present invention include, but are not limited to, liquid dispersions, gels, aerosols (pulmonary and nasal), ointments, creams, solid dose forms, *etc.* of nanoparticulate metaxalone. Lower dosages can be used because the small particle size of the metaxalone particles ensure greater absorption, and in the case of bioadhesive nanoparticulate metaxalone compositions, the metaxalone is retained at the desired site of application for a longer period of time as compared to conventional metaxalone dosage forms.

In one embodiment of the invention, the therapeutically effective amount of the nanoparticulate metaxalone compositions is 1/6, 1/5, ¼, 1/3^{rd}, or ½ of the therapeutically effective amount of a non-nanoparticulate metaxalone composition, such as SKELAXIN^{®}.

Such lower doses are preferred as they may decrease or eliminate adverse effects of the drug. In addition, such lower doses decrease the cost of the dosage form and may increase patient compliance.

### 3. Increased Bioavailability

The nanoparticulate metaxalone compositions of the invention may preferably exhibit increased bioavailability and require smaller doses as compared to prior non-nanoparticulate metaxalone compositions, such as SKELAXIN^{®}, administered at the same dose.

Any drug, including metaxalone, can have adverse side effects. Thus, lower doses of metaxalone which can achieve the same or better therapeutic effects as those observed with larger doses of non-nanoparticulate metaxaione compositions, such as SKELAXIN^{®}, are desired. Such lower doses may be realized with the nanoparticulate metaxalone compositions of the invention because the nanoparticulate metaxalone compositions may exhibit greater bioavailability as compared to non-nanoparticulate metaxalone formulations, which means that smaller dose of metaxalone are likely required to obtain the desired therapeutic effect.

As shown in the examples below (*e*.*g*., Table 4), two exemplary nanoparticulate metaxalone compositions exhibited an increase in AUC of about 859% and about 1153% over the AUC for the non-nanoparticulate metaxalone formulation, SKELAXIN^{®}.

### 4. Pharmacokinetic Profiles of the Nanoparticulate Metaxalone Compositions of the Invention

The invention also preferably provides metaxalone compositions having a desirable pharmacokinetic profile when administered to mammalian subjects. The desirable pharmacokinetic profile of the metaxalone compositions preferably includes, but is not limited to: (1) a Tₘₐₓ for metaxalone; when assayed in the plasma of a mammalian subject following administration, that is preferably less than the Tₘₐₓ for a non-nanoparticulate metaxalone formulation (*e*.*g*., SKELAXIN^{®}), administered at the same dosage; (2) a Cₘₐₓ for metaxalone, when assayed in the plasma of a mammalian subject following administration, that is preferably greater than the Cₘₐₓ for a non-nanoparticulate metaxalone formulation (*e*.*g*., SKELAXIN^{®}), administered at the same dosage; and/or (3) an AUC for metaxalone, when assayed in the plasma of a mammalian subject following administration, that is preferably greater than the AUC for a non-nanoparticulate metaxalone formulation (*e*.*g*., SKELAXIN^{®}), administered at the same dosage.

The desirable pharmacokinetic profile, as used herein, is the pharmacokinetic profile measured after the initial dose of metaxalone. The compositions can be formulated in any way as described below and as known to those of skill in the art.

A preferred metaxalone composition of the invention exhibits in comparative pharmacokinetic testing with a non-nanoparticulate metaxalone formulation (*e*.*g*., SKELAXIN^{®}), administered at the same dosage, a Tₘₐₓ not greater than about 90%, not greater than about 80%, not greater than about 70%, not greater than about 60%, not greater than about 50%, not greater than about 30%, not greater than about 25%, not greater than about 20%, not greater than about 15%, not greater than about 10%, or not greater than about 5% of the Tₘₐₓ exhibited by the non-nanoparticulate metaxalone formulation.

A preferred metaxalone composition of the invention exhibits in comparative pharmacokinetic testing with a non-nanoparticulate metaxalone formulation of (*e*.*g*., SKELAXIN^{®}), administered at the same dosage, a Cₘₐₓ which is at least about 50%, at least about 100%, at least about 200%, at least about 300%, at least about 400%, at least about 500%, at least about 600%, at least about 700%, at least about 800%, at least about 900%, at least about 1000%, at least about 1100%, at least about 1200%, at least about 1300%, at least about 1400%, at least about 1500%, at least about 1600%, at least about 1700%, at least about 1800%, or at least about 1900% greater than the Cₘₐₓ exhibited by the non-nanoparticulate metaxalone formulation.

A preferred metaxalone composition of the invention exhibits in comparative pharmacokinetic testing with a non-nanoparticulate metaxalone formulation (e.g., SKELAXIN^{®}), administered at the same dosage, an AUC which is at least about 25%, at least about 50%, at least about 75%, at least about 100%, at least about 125%, at least about 150%, at least about 175%, at least about 200%, at least about 225%, at least about 250%, at least about 275%, at least about 300%, at least about 350%, at least about 400%, at least about 450%, at least about 500%, at least about 550%, at least about 600%, at least about 750%, at least about 700%, at least about 750%, at least about 800%, at least about 850%, at least about 900%, at least about 950%, at least about 1000%, at least about 1050%, at least about 1100%, at least about 1150%, or at least about 1200% greater than the AUC exhibited by the non-nanoparticulate metaxalone formulation.

Any formulation giving the desired pharmacokinetic profile is suitable for administration according to the present methods. Exemplary types of formulations giving such profiles are liquid dispersions, gels, aerosols, ointments, creams, solid dose forms, *etc.* of nanoparticulate metaxalone.

### 5. The Pharmacokinetic Profiles of the Nanoparticulate Metaxalone Compositions of the Invention are Preferably not Substantially Affected by the Fed or Fasted State of the Subject Ingesting the Compositions

The invention encompasses nanoparticulate metaxalone compositions wherein preferably the pharmacokinetic profile of the metaxalone is not substantially affected by the fed or fasted state of a subject ingesting the composition. This means that there is no substantial difference in the quantity of metaxalone absorbed or the rate of metaxalone absorption when the nanoparticulate metaxalone compositions are administered in the fed versus the fasted state. Thus, the nanoparticulate metaxalone compositions of the invention can substantially eliminate the effect of food on the pharmacokinetics of metaxalone.

In another embodiment of the invention, the pharmacokinetic profile of the metaxalone compositions of the invention, when administered to a mammal in a fasted state, is bioequivalent to the pharmacokinetic profile of the same metaxalone composition administered at the same dosage, when administered to a mammal in a fed state. "Bioequivalency" is preferably established by a 90% Confidence Interval (CI) of between 0.80 and 1.25 for both Cₘₐₓ and AUC under U.S. Food and Drug Administration (USFDA) regulatory guidelines, or a 90% CI for AUC of between 0.80 to 1.25 and a 90% CI for Cₘₐₓ of between 0.70 to 1.43 under the European Medicines Evaluation Agency (EMEA) regulatory guidelines (Tₘₐₓ is not relevant for bioequivalency determinations under USFDA and EMEA regulatory guidelines).

Preferably the difference in AUC (*e*.*g*., absorption) of the nanoparticulate metaxalone composition of the invention, when administered in the fed versus the fasted state, is less than about 100%, less than about 90%, less than about 80%, less than about 70%, less than about 60%, less than about 50%, less than about 40%, less than about 35%, less than about 30%, less than about 25%, less than about 20%, less than about 15%, less than about 10%, less than about 5%, or less than about 3%.

In addition, preferably the difference in Cₘₐₓ of the nanoparticulate metaxalone composition of the invention, when administered in the fed versus the fasted state, is less than about 100%, less than about 90%, less than about 80%, less than about 70%, less than about 60%, less than about 50%, less than about 40%, less than about 35%, less than about 30%, less than about 25%, less than about 20%, less than about 15%, less than about 10%, less than about 5%, or less than about 3%.

Finally, preferably the difference in the Tₘₐₓ of the nanoparticulate metaxalone compositions of the invention, when administered in the fed versus the fasted state, is less than about 100%, less than about 90%, less than about 80%, less than about 70%, less than about 60%, less than about 50%, less than about 40%, less than about 30%, less than about 20%, less than about 15%, less than about 10%, less than about 5%, less than about 3%, or essentially no difference.

Benefits of a dosage form which substantially eliminates the effect of food include an increase in subject convenience, thereby increasing subject compliance, as the subject does not need to ensure that they are taking a dose either with or without food.

### 6. Redispersibility Profiles of the Nanoparticulate Metaxalone Compositions of the Invention

An additional feature of the nanoparticulate metaxalone compositions of the invention is that the compositions redisperse such that the effective average particle size of the redispersed metaxalone particles is less than about 2 microns. This is significant, as if upon administration the nanoparticulate metaxalone particles present in the compositions of the invention did not redisperse to a substantially nanoparticulate particle size, then the dosage form may lose the benefits afforded by formulating metaxalone into a nanoparticulate particle size.

This is because nanoparticulate metaxalone compositions benefit from the small particle size of metaxalone; if the nanoparticulate metaxalone particles do not redisperse into the small particle sizes upon administration, then "clumps" or agglomerated metaxalone particles are formed. With the formation of such agglomerated particles, the bioavailability of the dosage form may fall.

Moreover, the nanoparticulate metaxalone compositions of the invention exhibit dramatic redispersion of the metaxalone particles upon administration to a mammal, such as a human or animal, as demonstrated by reconstitution in a biorelevant aqueous media. Such biorelevant aqueous media can be any aqueous media that exhibit the desired ionic strength and pH, which form the basis for the biorelevance of the media. The desired pH and ionic strength are those that are representative of physiological conditions found in the human body. Such biorelevant aqueous media can be, for example, aqueous electrolyte solutions or aqueous solutions of any salt, acid, or base, or a combination thereof, which exhibit the desired pH and ionic strength.

Biorelevant pH is well known in the art. For example, in the stomach, the pH ranges from slightly less than 2 (but typically greater than 1) up to 4 or 5. In the small intestine the pH can range from 4 to 6, and in the colon it can range from 6 to 8. Biorelevant ionic strength is also well known in the art. Fasted state gastric fluid has an ionic strength of about 0.1M while fasted state intestinal fluid has an ionic strength of about 0.14. *See e*.*g*., Lindahl et al., "Characterization of Fluids from the Stomach and Proximal Jejunum in Men and Women," Pharm. Res., 14 (4): 497-502 (1997).

It is believed that the pH and ionic strength of the test solution is more critical than the specific chemical content. Accordingly, appropriate pH and ionic strength values can be obtained through numerous combinations of strong acids, strong bases, salts, single or multiple conjugate acid-base pairs (*i*.*e*., weak acids and corresponding salts of that acid), monoprotic and polyprotic electrolytes, *etc*.

Representative electrolyte solutions can be, but are not limited to, HCl solutions, ranging in concentration from about 0.001 to about 0.1 M, and NaCl solutions, ranging in concentration from about 0.001 to about 0.1 M, and mixtures thereof. For example, electrolyte solutions can be, but are not limited to, about 0.1 M HCl or less, about 0.01 M HCl or less, about 0.001 M HCl or less, about 0.1 M NaCl or less, about 0.01 M NaCl or less, about 0.001 M NaCl or less, and mixtures thereof. Of these electrolyte solutions, 0.01 M HCl and/or 0.1 M NaCl, are most representative of fasted human physiological conditions, owing to the pH and ionic strength conditions of the proximal gastrointestinal tract.

Electrolyte concentrations of 0.001 M HCl, 0.01 M HCl, and 0.1 M HCl correspond to pH 3, pH 2, and pH 1, respectively. Thus, a 0.01 M HCl solution simulates typical acidic conditions found in the stomach. A solution of 0.1 M NaCl provides a reasonable approximation of the ionic strength conditions found throughout the body, including the gastrointestinal fluids, although concentrations higher than 0.1 M may be employed to simulate fed conditions within the human GI tract.

Exemplary solutions of salts, acids, bases or combinations thereof, which exhibit the desired pH and ionic strength, include but are not limited to phosphoric acid/phosphate salts + sodium, potassium and calcium salts of chloride, acetic acid/acetate salts + sodium, potassium and calcium salts of chloride, carbonic acid/bicarbonate salts + sodium, potassium and calcium salts of chloride, and citric acid/citrate salts + sodium, potassium and calcium salts of chloride.

In other embodiments of the invention, the redispersed metaxalone particles of the invention (redispersed in an aqueous, biorelevant, or any other suitable media) have an effective average particle size of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods.

By "an effective average particle size of less than about 2000 nm" it is meant that at least 50% of the metaxalone particles have a particle size of less than the effective average, by weight, *i*.*e*., less than about 2000 nm, 1900 nm, 1800 nm, etc., when measured by the above-noted techniques. Preferably, at least about 70%, about 90%, about 95%, or about 99% of the metaxalone particles have a particle size of less than the effective average, *i*.*e*., less than about 2000 nm, 1900 nm, 1800 nm, 1700 nm, *etc.*

Redispersibility can be tested using any suitable means known in the art. *See e*.*g*., the example sections of U.S. Patent No. 6,375,986 for "Solid Dose Nanoparticulate Compositions Comprising a Synergistic Combination of a Polymeric Surface Stabilizer and Dioctyl Sodium Sulfosuccinate."

### 7. Bioadhesive Nanoparticulate Metaxalone Compositions

Bioadhesive nanoparticulate metaxalone compositions of the invention comprise at least one cationic surface stabilizer, which are described in more detail below. Bioadhesive formulations of metaxalone exhibit exceptional bioadhesion to biological surfaces, such as mucous.

In the case of bioadhesive nanoparticulate metaxalone compositions, the term "bioadhesion" is used to describe the adhesion between the nanoparticulate metaxalone compositions and a biological substrate (*i*.*e*., gastrointestinal mucin, lung tissue, nasal mucosa, *etc*.). *See e*.*g*., U.S. Patent No. 6,428,814 for "Bioadhesive Nanoparticulate Compositions Having Cationic Surface Stabilizers," which is specifically incorporated by reference.

The bioadhesive metaxalone compositions of the invention are useful in any situation in which it is desirable to apply the compositions to a biological surface. The bioadhesive metaxalone compositions preferably coat the targeted surface in a continuous and uniform film which is invisible to the naked human eye.

A bioadhesive nanoparticulate metaxalone composition slows the transit of the composition, and some metaxalone particles would also most likely adhere to tissue other than the mucous cells and therefore give a prolonged exposure to metaxalone, thereby increasing absorption and the bioavailability of the administered dosage.

### 8. Low Viscosity

A liquid dosage form of a conventional microcrystalline or non-nanoparticulate metaxalone composition would be expected to be a relatively large volume, highly viscous substance which would not be well accepted by patient populations. Moreover, viscous solutions can be problematic in parenteral administration because these solutions require a slow syringe push and can stick to tubing. In addition, conventional formulations of poorly water-soluble active agents, such as metaxalone, tend to be unsafe for intravenous administration techniques, which are used primarily in conjunction with highly water-soluble substances.

Liquid dosage forms of the nanoparticulate metaxalone compositions of the invention provide significant advantages over a liquid dosage form of a conventional metaxalone microcrystalline compound. The low viscosity and silky texture of liquid dosage forms of the nanoparticulate metaxalone compositions of the invention result in advantages in both preparation and use. These advantages include, for example:
(1) better subject compliance due to the perception of a lighter formulation which is easier to consume and digest; (2) ease of dispensing because one can use a cup or a syringe; (3) potential for formulating a higher concentration of metaxalone resulting in a smaller dosage volume and thus less volume for the subject to consume; and (4) easier overall formulation concerns.

Liquid metaxalone dosage forms which are easier to consume are especially important when considering juvenile patients, terminally ill patients, and elderly patients. Viscous or gritty formulations, and those that require a relatively large dosage volume, are not well tolerated by these patient populations. Liquid oral dosage forms can be particularly preferably for patient populations who have difficulty consuming tablets, such as infants and the elderly.

The viscosities of liquid dosage forms of nanoparticulate metaxalone according to the invention are preferably less than about 1/200, less than about 1/175, less than about 1/150, less than about 1/125, less than about 1/100, less than about 1/75, less than about 1/50, or less than about 1/25 of a liquid oral dosage form of a non-nanoparticulate metaxalone composition, at about the same concentration per ml of metaxalone.

Typically the viscosity of liquid nanoparticulate metaxalone dosage forms of the invention, at a shear rate of 0.1 (1/s), is from about 2000 mPa s to about 1 mPa s, from about 1900 mPa·s to about 1 mPa·s, from about 1800 mPa·s to about 1 mPa·s, from about 1700 mPa·s to about 1 mPa·s, from about 1600 mPa·s to about 1 mPa·s, from about 1500 mPa·s to about 1 mPa·s, from about 1400 mPa·s to about 1 mPa·s, from about 1300 mPa·s to about 1 mPa·s, from about 1200 mPa·s to about 1 mPa·s, from about 1100 mPa·s to about 1 mPa·s, from about 1000 mPa·s to about 1 mPa·s, from about 900 mPa·s to about 1 mPa·s, from about 800 mPa·s to about 1 mPa·s, from about 700 mPa·s to about 1 mPa·s, from about 600 mPa·s to about 1 mPa·s, from about 500 mPa·s to about 1 mPa·s, from about 400 mPa·s to about 1 mPa·s, from about 300 mPa·s to about 1 mPa·s, from about 200 mPa·s to about 1 mPa·s, from about 175 mPa·s to about 1 mPa·s, from about 150 mPa·s to about 1 mPa·s, from about 125 mPa·s to about 1 mPa·s, from about 100 mPa·s to about 1 mPa·s, from about 75 mPa·s to about 1 mPa·s, from about 50 mPa·s to about 1 mPa·s, from about 25 mPa·s to about 1 mPa·s, from about 15 mPa·s to about 1 mPa·s, from about 10 mPa·s to about 1 mPa·s, or from about 5 mPa·s to about 1 mPa·s. Such a viscosity is much more attractive for subject consumption and may lead to better overall subject compliance.

Viscosity is concentration and temperature dependent. Typically, a higher concentration results in a higher viscosity, while a higher temperature results in a lower viscosity. Viscosity as defined above refers to measurements taken at about 20°C. (The viscosity of water at 20°C is 1 mPa s.) The invention encompasses equivalent viscosities measured at different temperatures.

Another important aspect of the invention is that the nanoparticulate metaxalone compositions of the invention, formulated into a liquid dosage form, are not turbid. "Turbid," as used herein refers to the property of particulate matter that can be seen with the naked eye or that which can be felt as "gritty." The nanoparticulate metaxalone compositions of the invention, formulated into a liquid dosage form, can be poured out of or extracted from a container as easily as water, whereas a liquid dosage form of a non-nanoparticulate or solubilized metaxalone is expected to exhibit notably more "sluggish" characteristics.

The liquid formulations of this invention can be formulated for dosages in any volume but preferably equivalent or smaller volumes than a liquid dosage form of a non-nanoparticulate metaxalone composition.

### 9. Sterile Filtered Nanoparticulate Metaxalone Compositions

The nanoparticulate metaxalone compositions of the invention can be sterile filtered. This obviates the need for heat sterilization, which can harm or degrade metaxalone, as well as result in crystal growth and particle aggregation.

Sterile filtration can be difficult because of the required small particle size of the composition. Filtration is an effective method for sterilizing homogeneous solutions when the membrane filter pore size is less than or equal to about 0.2 microns (200 nm) because a 0.2 micron filter is sufficient to remove essentially all bacteria. Sterile filtration is normally not used to sterilize suspensions of micron-sized metaxalone because the metaxalone particles are too large to pass through the membrane pores.

A sterile nanoparticulate metaxalone dosage form is particularly useful in treating immunocompromised patients, infants or juvenile patients, and the elderly, as these patient groups are the most susceptible to infection caused by a non-sterile liquid dosage form.

Because the nanoparticulate metaxalone compositions of the invention, formulated into a liquid dosage form, can be sterile filtered, and because the compositions can have a very small metaxalone effective average particle size, the compositions are suitable for parenteral administration.

### 10. Combination Pharmacokinetic Profile Compositions

In yet another embodiment of the invention, a first nanoparticulate metaxalone composition providing a desired pharmacokinetic profile is co-administered, sequentially administered, or combined with at least one other metaxalone composition that generates a desired different pharmacokinetic profile. More than two metaxalone compositions can be co-administered, sequentially administered, or combined. While the first metaxalone composition has a nanoparticulate particle size, the additional one or more metaxalone compositions can be nanoparticulate, solubilized, or have a microparticulate particle size.

For example, a first metaxalone composition can have a nanoparticulate particle size, conferring a short Tₘₐₓ and typically a higher Cₘₐₓ. This first metaxalone composition can be combined, co-administered, or sequentially administered with a second composition comprising: (1) metaxalone having a larger (but still nanoparticulate as defined herein) particle size, and therefore exhibiting slower absorption, a longer Tₘₐₓ, and typically a lower Cₘₐₓ; or (2) a microparticulate or solubilized metaxalone composition, exhibiting a longer Tₘₐₓ, and typically a lower Cₘₐₓ.

The second, third, fourth, etc., metaxalone compositions can differ from the first, and from each other, for example: (1) in the effective average particle sizes of metaxalone; or (2) in the dosage of metaxalone. Such a combination composition can reduce the dose frequency required.

If the second metaxalone composition has a nanoparticulate particle size, then preferably the metaxalone particles of the second composition have at least one surface stabilizer associated with the surface of the drug particles. The one or more surface stabilizers can be the same as or different from the surface stabilizer(s) present in the first metaxalone composition.

Preferably where co-administration of a "fast-acting" formulation and a "longer-lasting" formulation is desired, the two formulations are combined within a single composition, for example a dual-release composition.

### 11. Combination Active Agent Compositions

The invention encompasses the nanoparticulate metaxalone compositions of the invention formulated or co-administered with one or more non-metaxalone active agents. Methods of using such combination compositions are also encompassed by the invention. The non-metaxalone active agents can be present in a crystalline phase, an amorphous phase, a semi-crystalline phase, a semi-amorphous phase, or a mixture thereof.

The compound to be administered in combination with a nanoparticulate metaxalone composition of the invention can be formulated separately from the nanoparticulate metaxalone composition or co-formulated with the nanoparticulate metaxalone composition. Where a nanoparticulate metaxalone composition is co-formulated with a second active agent, the second active agent can be formulated in any suitable manner, such as immediate-release, rapid-onset, sustained-release, or dual-release form.

Such non-metaxalone active agents can be, for example, a therapeutic agent. A therapeutic agent can be a pharmaceutical agent, including a biologic. The active agent can be selected from a variety of known classes of drugs, including, for example, amino acids, proteins, peptides, nucleotides, anti-obesity drugs, central nervous system stimulants, carotenoids, corticosteroids, elastase inhibitors, anti-fungals, oncology therapies, anti-emetics, analgesics, cardiovascular agents, anti-inflammatory agents, such as NSAIDs and COX-2 inhibitors, anthelmintics, anti-arrhythmic agents, antibiotics (including penicillins), anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives (hypnotics and neuroleptics), astringents, alpha-adrenergic receptor blocking agents, beta-adrenoceptor blocking agents, blood products and substitutes, cardiac inotropic agents, contrast media, corticosteroids, cough suppressants (expectorants and mucolytics), diagnostic agents, diagnostic imaging agents, diuretics, dopaminergics (antiparkinsonian agents), haemostatics, immunological agents, lipid regulating agents, muscle relaxants, parasympathomimetics, parathyroid calcitonin and biphosphonates, prostaglandins, radio-pharmaceuticals, sex hormones (including steroids), anti-allergic agents, stimulants and anoretics, sympathomimetics, thyroid agents, vasodilators, and xanthines.

Examples of representative active agents useful in this invention include, but are not limited to, acyclovir, alprazolam, altretamine, amiloride, amiodarone, benztropine mesylate, bupropion, cabergoline, candesartan, cerivastatin, chlorpromazine, ciprofloxacin, cisapride, clarithromycin, clonidine, clopidogrel, cyclobenzaprine, cyproheptadine, delavirdine, desmopressin, diltiazem, dipyridamole, dolasetron, enalapril maleate, enalaprilat, famotidine, felodipine, furazolidone, glipizide, irbesartan, ketoconazole, lansoprazole, loratadine, loxapine, mebendazole, mercaptopurine, milrinone lactate, minocycline, mitoxantrone, nelfinavir mesylate, nimodipine, norfloxacin, olanzapine, omeprazole, penciclovir, pimozide, tacolimus, quazepam, raloxifene, rifabutin, rifampin, risperidone, rizatriptan, saquinavir, sertraline, sildenafil, acetyl-sulfisoxazole, temazepam, thiabendazole, thioguanine, trandolapril, triamterene, trimetrexate, troglitazone, trovafloxacin, verapamil, vinblastine sulfate, mycophenolate, atovaquone, atovaquone, proguanil, ceftazidime, cefuroxime, etoposide, terbinafine, thalidomide, fluconazole, amsacrine, dacarbazine, teniposide, and acetylsalicylate.

A description of these classes of active agents and a listing of species within each class can be found in Martindale's The Extra Pharmacopoeia, 31st Edition (The Pharmaceutical Press, London, 1996), specifically incorporated by reference. The active agents are commercially available and/or can be prepared by techniques known in the art.

Exemplary nutraceuticals or dietary supplements include, but are not limited to, lutein, folic acid, fatty acids (*e*.*g*., DHA and ARA), fruit and vegetable extracts, vitamin and mineral supplements, phosphatidylserine, lipoic acid, melatonin, glucosamine/chondroitin, Aloe Vera, Guggul, glutamine, amino acids (*e*.*g*., arginine, isoleucine, leucine, lysine, methionine, phenylanine, threonine, tryptophan, and valine), green tea, lycopene, whole foods, food additives, herbs, phytonutrients, antioxidants, flavonoid constituents of fruits, evening primrose oil, flax seeds, fish and marine animal oils, and probiotics. Nutraceuticals and dietary supplements also include bio-engineered foods genetically engineered to have a desired property, also known as "pharmafoods."

Exemplary nutraceuticals and dietary supplements are disclosed, for example, in Roberts et al., Nutraceuticals: The Complete Encyclopedia of Supplements, Herbs, Vitamins, and Healing Foods (American Nutraceutical Association, 2001). Dietary supplements and nutraceuticals are also disclosed in Physicians' Desk Reference for Nutritional Supplements, 1st Ed. (2001) and The Physicians' Desk Reference for Herbal Medicines, 1st Ed. (2001), both of which are also incorporated by reference. A nutraceutical or dietary supplement, also known as a phytochemical or functional food, is generally any one of a class of dietary supplements, vitamins, minerals, herbs, or healing foods that have medical or pharmaceutical effects on the body.

In a particularly preferred embodiment of the invention, the nanoparticulate metaxalone composition is combined with at least one analgesic. Useful analgesics include, for example, NSAIDS and COX-2 inhibitors.

Examples of NSAIDS include, but are not limited to, suitable nonacidic and acidic compounds. Suitable nonacidic compounds include, for example, nabumetone, tiaramide, proquazone, bufexamac, flumizole, epirazole, tinoridine, timegadine, and dapsone.

Suitable acidic compounds include carboxylic acids and enolic acids. Suitable carboxylic acid NSAIDs include, for example: (1) salicylic acids and esters thereof, such as aspirin, diflunisal, benorylate, and fosfosal; (2) acetic acids, including phenylacetic acids such as diclofenac, alclofenac and fenclofenac; (3) carbo- and heterocyclic acetic acids such as etodolac, indomethacin, sulindac, tolmetin, fentiazac, and tilomisole; (4) propionic acids, such as carprofen, fenbufen, flurbiprofen, ketoprofen, oxaprozin, suprofen, tiaprofenic acid, ibuprofen, naproxen, fenoprofen, indoprofen, pirprofen; and (5) fenamic acids, such as flufenamic, mefenamic, meclofenamic and niflumic.

Suitable enolic acid NSAIDs include, for example: (1) pyrazolones such as oxyphenbutazone, phenylbutazone, apazone, and feprazone; and (2) oxicams such as piroxicam, sudoxicam, isoxicam, and tenoxicam.

Currently identified COX-2 inhibitors include, but are not limited to, celecoxib (SC-58635, CELEBREX^{®}, Pharmacia/Searle & Co.), rofecoxib (MK-966, L-748731, VIOXX^{®}, Merck & Co.), meloxicam (MOBIC^{®}, co-marketed by Abbott Laboratories, Chicago, IL, and Boehringer Ingelheim Pharmaceuticals), valdecoxib (BEXTRA^{®}, G.D. Searle & Co.), parecoxib (G.D. Searle & Co.), etoricoxib (MK-663; Merck), SC-236 (chemical name of 4-[5-(4-chlorophenyl)-3-(trifluoromethyl)-1H-pyrazol-1-yl)] benzenesulfonamide; G.D. Searle & Co., Skokie, IL); NS-398 (N-(2-cyclohexyloxy-4-nitrophenyl)methane sulfonamide; Taisho Pharmaceutical Co., Ltd., Japan); SC-58125 (methyl sulfone spiro(2.4)hept-5-ene I; Pharmacia/Searle & Co.); SC-57666 (Pharmacia/Searle & Co.); SC-558 (Pharmacia/Searle & Co.); SC-560 (Pharmacia/Searle & Co.); etodolac (Lodine^{®}, Wyeth-Ayerst Laboratories, Inc.); DFU (5,5-dimethyl-3-(3-fluorophenyl)-4-(4-methylsulfonyl)phenyl 2(5H)-furanone); monteleukast (MK-476), L-745337 ((5-methanesulphonamide-6-(2,4-difluorothio-phenyl)-1-indanone), L-761066, L-761000, L-748780 (all Merck & Co.); DUP-697 (5-Bromo-2-(4-fluorophenyl)-3-(4-(methylsulfonyl)phenyl; DuPont Merck Pharmaceutical Co.); PGV 20229 (1-(7-tert.-butyl-2,3-dihydro-3,3-dimethylbenzo(b)furan-5-yl)-4-cyclopropylbutan-1-one; Procter & Gamble Pharmaceuticals); iguratimod (T-614; 3-formylamino-7-methylsulfonylamino-6-phenoxy-4H-1- benzopyran-4-one; Toyama Corp., Japan); BF 389 (Biofor, USA); CL 1004 (PD 136095), PD 136005, PD 142893, PD 138387, and PD 145065 (all Parke-Davis/Warner-Lambert Co.); flurbiprofen (ANSAID^{®}; Pharmacia & Upjohn); nimesulide (NIM-03, R 805, 4 nitro 2 phenoxymethane sulfonanilide, MESULID^{®}; Hisamitsu, Japan); nabumetone (FELAFEN^{®}; SmithKline Beecham, plc); flosulide (CGP 28238; Novartis/Ciba Geigy); piroxicam (FELDANE^{®}; Pfizer); diclofenac (VOLTAREN^{®} and CATAFLAM^{®}, Novartis); lumiracoxib (COX-189; Novartis); D 1367 (Celltech Chiroscience, plc); R 807 (3 benzoyldifluoromethane sulfonanilide, diflumidone); JTE-522 (Japan Tobacco, Japan); FK-3311 (4'-Acetyl-2'-(2,4-difluorophenoxy)methanesulfonanilide), FK 867, FR 140423, and FR 115068 (all Fujisawa, Japan); GR 253035 (Glaxo Wellcome); RWJ 63556 (Johnson & Johnson); RWJ 20485 (Johnson & Johnson); ZK 38997 (Schering); S 2474 ((E)-(5)-(3,5-di-tert-butyl-4-hydroxybenzylidene)-2-ethyl-1,2-isothiazolidine-1,1-dioxide indomethacin; Shionogi & Co., Ltd., Japan); zomepirac analogs, such as RS 57067 and RS 104897 (Hoffmann La Roche); RS 104894 (Hoffmann La Roche); SC 41930 (Monsanto); pranlukast (SB 205312, Ono-1078, ONON^{®}, ULTAIR^{®}; SmithKline Beecham); SB 209670 (SmithKline Beecham); APHS (heptinylsulfide);

### 12. Miscellaneous Benefits of the Nanoparticulate Metaxalone Compositions of the Invention

The nanoparticulate metaxalone compositions preferably exhibit an increased rate of dissolution as compared to microcrystalline or non-nanoparticulate forms of metaxalone. In addition, the nanoparticulate metaxalone compositions preferably exhibit improved performance characteristics for oral, intravenous, subcutaneous, or intramuscular injection, such as higher dose loading and smaller tablet or liquid dose volumes. Moreover, the nanoparticulate metaxalone compositions of the invention do not require organic solvents or pH extremes.

### B. Metaxalone Compositions

The invention provides compositions comprising nanoparticulate metaxalone particles and at least one surface stabilizer. The surface stabilizers are preferably associated with the surface of the metaxalone particles. Surface stabilizers useful herein do not chemically react with the metaxalone particles or itself. Preferably, individual molecules of the surface stabilizer are essentially free of intermolecular cross-linkages. The compositions can comprise two or more surface stabilizers.

The present invention also includes nanoparticulate metaxalone compositions together with one or more non-toxic physiologically acceptable carriers, adjuvants, or vehicles, collectively referred to as carriers. The compositions can be formulated for parenteral injection (*e*.*g*., intravenous, intramuscular, or subcutaneous), oral administration (in solid, liquid, or aerosol (*i*.*e*., pulmonary) form), vaginal, nasal, rectal, ocular, local (powders, creams, ointments or drops), buccal, intracisternal, intraperitoneal, topical administration, and the like.

### 1. Metaxalone Particles

As used herein, "metaxalone" means 5-[(3,4-dimethylphenoxy)methyl]-2-oxazolidinone or a salt thereof having the following chemical structure:

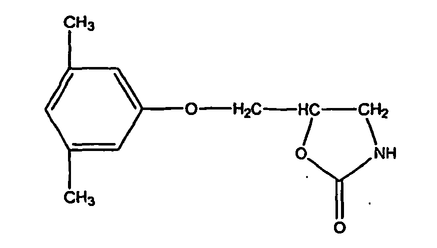

### Derivatives of metaxalone are also encompassed by the term "metaxalone."

Metaxalone is a skeletal muscle relaxant used to relieve the pain of muscle injuries, spasms, sprains, and strains. The mechanism of action of metaxalone in humans has not been established, but may be due to general central nervous system depression. It has no direct action on the contractile mechanism of striated muscle, the motor end plate, or the nerve fiber. The drug does not directly relax tense skeletal muscles in man. *See* The Physician's Desk Reference, 57th Edition, p. 1274 (Thompson PDR, Montvale NJ, 2003).

Metaxalone can be in a crystalline phase, an amorphous phase, a semi-crystalline phase, a semi-amorphous phase, or a mixtures thereof.

### 2. Surface Stabilizers

The choice of a surface stabilizer for metaxalone is non-trivial and required extensive experimentation to realize a desirable formulation. Accordingly, the present invention is directed to the surprising discovery that metaxalone nanoparticulate compositions can be made.

Combinations of more than one surface stabilizer can be used in the invention. Useful surface stabilizers which can be employed in the invention include, but are not limited to, known organic and inorganic pharmaceutical excipients. Such excipients include various polymers, low molecular weight oligomers, natural products, and surfactants. Surface stabilizers include nonionic, cationic, zwitterionic, and ionic surfactants.

Representative examples of other useful surface stabilizers include hydroxypropyl methylcellulose, hydroxypropylcellulose, polyvinylpyrrolidone, sodium lauryl sulfate, dioctylsulfosuccinate, gelatin, casein, lecithin (phosphatides), dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (*e*.*g*., macrogol ethers such as cetomacrogol 1000), polyoxyethylene castor oil derivatives, polyoxyethylene sorbitan fatty acid esters (*e*.*g*., the commercially available Tweens^{®} such as *e*.*g*., Tween 20^{®} and Tween 80^{®} (ICI Speciality Chemicals)); polyethylene glycols (*e*.*g*., Carbowaxs 3550^{®} and 934^{®} (Union Carbide)), polyoxyethylene stearates, colloidal silicon dioxide, phosphates, carboxymethylcellulose calcium, carboxymethylcellulose sodium, methylcellulose, hydroxyethylcellulose, hydroxypropylmethylcellulose phthalate, noncrystalline cellulose, magnesium aluminium silicate, triethanolamine, polyvinyl alcohol (PVA), 4-(1,1,3,3-tetramethylbutyl)-phenol polymer with ethylene oxide and formaldehyde (also known as tyloxapol, superione, and triton), poloxamers (*e*.*g*., Pluronics F68^{®} and F108^{®}, which are block copolymers of ethylene oxide and propylene oxide); poloxamines (*e*.*g*., Tetronic 908^{®}, also known as Poloxamine 908^{®}, which is a tetrafunctional block copolymer derived from sequential addition of propylene oxide and ethylene oxide to ethylenediamine (BASF Wyandotte Corporation, Parsippany, N.J.)); Tetronic 1508^{®} (T-1508) (BASF Wyandotte Corporation), Tritons X-200^{®}, which is an alkyl aryl polyether sulfonate (Rohm and Haas); Crodestas F-110^{®}, which is a mixture of sucrose stearate and sucrose distearate (Croda Inc.); p-isononylphenoxypoly-(glycidol), also known as Olin-lOG^{®} or Surfactant 10-G^{®} (Olin Chemicals, Stamford, CT); Crodestas SL-40^{®} (Croda, Inc.); and SA9OHCO, which is C₁₈H₃₇CH₂(CON(CH₃)-CH₂(CHOH)₄(CH₂0H)₂ (Eastman Kodak Co.); decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; PEG-derivatized phospholipid, PEG- derivatized cholesterol, PEG-derivatized cholesterol derivative, PEG- derivatized vitamin A, PEG- derivatized vitamin E, lysozyme, random copolymers of vinyl pyrrolidone and vinyl acetate, and the like.

Depending upon the desired method of administration, bioadhesive formulations of nanoparticulate metaxalone can be prepared by selecting one or more cationic surface stabilizers that impart bioadhesive properties to the resultant composition. Useful cationic surface stabilizers are described below.

Examples of useful cationic surface stabilizers include, but are not limited to, polymers, biopolymers, polysaccharides, cellulosics, alginates, phospholipids, and nonpolymeric compounds, such as zwitterionic stabilizers, poly-n-methylpyridinium, anthryul pyridinium chloride, cationic phospholipids, chitosan, polylysine, polyvinylimidazole, polybrene, polymethylmethacrylate trimethylammoniumbromide bromide (PMMTMABr), hexyldesyltrimethylammonium bromide (HDMAB), polyvinylpyrrolidone-2-dimethylaminoethyl methacrylate dimethyl sulfate, 1,2 Dipalmitoyl-sn-Glycero-3-Phosphoethanolamine-N-[Amino(Polyethylene Glycol)2000] (sodium salt) (also known as DPPE-PEG(2000)-Amine Na) (Avanti Polar Lipids, Alabaster, Al), Poly(2-methacryloxyethyl trimethylammonium bromide) (Polysciences, Inc., Warrington, PA) (also known as S1001), poloxamines such as Tetronic 908^{®}, also known as Poloxamine 908^{®}, which is a tetrafunctional block copolymer derived from sequential addition of propylene oxide and ethylene oxide to ethylenediamine (BASF Wyandotte Corporation, Parsippany, N.J.), lysozyme, long-chain polymers such as alginic acid, carrageenan (FMC Corp.), and POLYOX (Dow, Midland, MI).

Other useful cationic stabilizers include, but are not limited to, cationic lipids, sulfonium, phosphonium, and quarternary ammonium compounds, such as stearyltrimethylammonium chloride, benzyl-di(2-chloroethyl)ethylammonium bromide, coconut trimethyl ammonium chloride or bromide, coconut methyl dihydroxyethyl ammonium chloride or bromide, decyl triethyl ammonium chloride, decyl dimethyl hydroxyethyl ammonium chloride or bromide, C₁₂₋₁₅dimethyl hydroxyethyl ammonium chloride or bromide, coconut dimethyl hydroxyethyl ammonium chloride or bromide, myristyl trimethyl ammonium methyl sulphate, lauryl dimethyl benzyl ammonium chloride or bromide, lauryl dimethyl (ethenoxy)₄ ammonium chloride or bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts and dialkyl-dimethylammonium salts, lauryl trimethyl ammonium chloride, ethoxylated alkyamidoalkyldialkylammonium salt and/or an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride and dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂, C₁₅, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride (ALIQUAT 336^{™}), POLYQUAT 10^{™}, tetrabutylammonium bromide, benzyl trimethylammonium bromide, choline esters (such as choline esters of fatty acids), benzalkonium chloride, stearalkonium chloride compounds (such as stearyltrimonium chloride and Distearyldimonium chloride), cetyl pyridinium bromide or chloride, halide salts of quatemized polyoxyethylalkylamines, MIRAPOL^{™} and ALKAQUAT^{™} (Alkaril Chemical Company), alkyl pyridinium salts; amines, such as alkylamines, dialkylamines, alkanolamines, polyethylenepolyamines, N,N-dialkylaminoalkyl acrylates, and vinyl pyridine, amine salts, such as lauryl amine acetate, stearyl amine acetate, alkylpyridinium salt, and alkylimidazolium salt, and amine oxides; imide azolinium salts; protonated quaternary acrylamides; methylated quaternary polymers, such as poly[diallyl dimethylammonium chloride] and poly-[N-methyl vinyl pyridinium chloride]; and cationic guar.

Such exemplary cationic surface stabilizers and other useful cationic surface stabilizers are described in J. Cross and E. Singer, Cationic Surfactants: Analytical and Biological Evaluation (Marcel Dekker, 1994); P. and D. Rubingh (Editor), Cationic Surfactants: Physical Chemistry (Marcel Dekker, 1991); and J. Richmond, Cationic Surfactants: Organic Chemistry, (Marcel Dekker, 1990).

Nonpolymeric cationic surface stabilizers are any nonpolymeric compound, such as benzalkonium chloride, a carbonium compound, a phosphonium compound, an oxonium compound, a halonium compound, a cationic organometallic compound, a quarternary phosphorous compound, a pyridinium compound, an anilinium compound, an ammonium compound, a hydroxylammonium compound, a primary ammonium compound, a secondary ammonium compound, a tertiary ammonium compound, and quarternary ammonium compounds of the formula NR₁R₂R₃R₄⁽⁺⁾. For compounds of the formula NR₁R₂R₃R₄⁽⁺⁾:
(i) none of R₁-R₄ are CH₃;
(ii) one of R₁-R₄ is CH₃;
(iii) three of R₁-R₄ are CH₃;
(iv) all of R₁-R₄ are CH₃;
(v) two of R₁-R₄ are CH₃, one of R₁-R₄ is C₆H₅CH₂, and one of R₁-R₄ is an alkyl chain of seven carbon atoms or less;
(vi) two of R₁-R₄ are CH₃, one of R₁-R₄ is C₆H₅CH₂, and one of R₁-R₄ is an alkyl chain of nineteen carbon atoms or more;
(vii) two of R₁-R₄ are CH₃ and one of R₁-R₄ is the group C₆H₅(CH₂)ₙ, where n>1;
(viii) two of R₁-R₄ are CH₃, one of R₁-R₄ is C₆H₅CH₂, and one of R₁-R₄ comprises at least one heteroatom;
(ix) two of R₁-R₄ are CH₃, one of R₁-R₄ is C₆H₅CH₂, and one of R₁-R₄ comprises at least one halogen;
(x) two of R₁-R₄ are CH₃, one of R₁-R₄ is C₆H₅CH₂, and one of R₁-R₄ comprises at least one cyclic fragment;
(xi) two of R₁-R₄ are CH₃ and one of R₁-R₄ is a phenyl ring; or
(xii) two of R₁-R₄ are CH₃ and two of R₁-R₄ are purely aliphatic fragments.

Such compounds include, but are not limited to, behenalkonium chloride, benzethonium chloride, cetylpyridinium chloride, behentrimonium chloride, lauralkonium chloride, cetalkonium chloride, cetrimonium bromide, cetrimonium chloride, cethylamine hydrofluoride, chlorallylmethenamine chloride (Quaternium-15), distearyldimonium chloride (Quaternium-5), dodecyl dimethyl ethylbenzyl ammonium chloride(Quaternium-14), Quaternium-22, Quaternium-26, Quaternium-18 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCI, iofetamine hydrochloride, meglumine hydrochloride, methylbenzethonium chloride, myrtrimonium bromide, oleyltrimonium chloride, polyquaternium-1, procainehydrochloride, cocobetaine, stearalkonium bentonite, stearalkoniumhectonite, stearyl trihydroxyethyl propylenediamine dihydrofluoride, tallowtrimonium chloride, and hexadecyltrimethyl ammonium bromide.

Most of these surface stabilizers are known pharmaceutical excipients and are described in detail in the Handbook of Pharmaceutical Excipients, published jointly by the American Pharmaceutical Association and The Pharmaceutical Society of Great Britain (The Pharmaceutical Press, 2000), specifically incorporated by reference.

The surface stabilizers are commercially available and/or can be prepared by techniques known in the art.

Preferred surface stabilizers include, but are not limited to, polyvinylpyrrolidone (PVP), docusate sodium (DOSS), and lysozyme. Particularly preferred is a combination of PVP and DOSS.

An example of a preferred composition is 35% metaxalone, 8.75% polyvinylpyrrolidone, and 0.35% DOSS.

### 3. Pharmaceutical Excipients

Pharmaceutical compositions according to the invention may also comprise one or more binding agents, filling agents, lubricating agents, suspending agents, sweeteners, flavoring agents, preservatives, buffers, wetting agents, disintegrants, effervescent agents, and other excipients. Such excipients are known in the art.

Examples of filling agents are lactose monohydrate, lactose anhydrous, and various starches; examples of binding agents are various celluloses and cross-linked polyvinylpyrrolidone, microcrystalline cellulose, such as Avicel^{®} PH101 and Avicel^{®} PH102, microcrystalline cellulose, and silicified microcrystalline cellulose (ProSolv SMCC^{™}).

Suitable lubricants, including agents that act on the flowability of the powder to be compressed, are colloidal silicon dioxide, such as Aerosil^{®} 200, talc, stearic acid, magnesium stearate, calcium stearate, and silica gel.

Examples of sweeteners are any natural or artificial sweetener, such as sucrose, xylitol, sodium saccharin, cyclamate, aspartame, and acsulfame. Examples of flavoring agents are Magnasweet^{®} (trademark of MAFCO), bubble gum flavor, and fruit flavors, and the like.

Examples of preservatives are potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quarternary compounds such as benzalkonium chloride.

Suitable diluents include pharmaceutically acceptable inert fillers, such as microcrystalline cellulose, lactose, dibasic calcium phosphate, saccharides, and/or mixtures of any of the foregoing. Examples of diluents include microcrystalline cellulose, such as Avicel^{®} PH101 and Avicel^{®} PH102; lactose such as lactose monohydrate, lactose anhydrous, and Pharmatose^{®} DCL21; dibasic calcium phosphate such as Emcompress^{®}; mannitol; starch; sorbitol; sucrose; and glucose.

Suitable disintegrants include lightly crosslinked polyvinyl pyrrolidone, corn starch, potato starch, maize starch, and modified starches, croscarmellose sodium, cross-povidone, sodium starch glycolate, and mixtures thereof.

Examples of effervescent agents are effervescent couples such as an organic acid and a carbonate or bicarbonate. Suitable organic acids include, for example, citric, tartaric, malic, fumaric, adipic, succinic, and alginic acids and anhydrides and acid salts. Suitable carbonates and bicarbonates include, for example, sodium carbonate, sodium bicarbonate, potassium carbonate, potassium bicarbonate, magnesium carbonate, sodium glycine carbonate, L-lysine carbonate, and arginine carbonate. Alternatively, only the sodium bicarbonate component of the effervescent couple may be present.

### 4. Nanoparticulate Metaxalone Particle Size

As used herein, particle size is determined on the basis of the weight average particle size as measured by conventional particle size measuring techniques well known to those skilled in the art. Such techniques include, for example, sedimentation field flow fractionation, photon correlation spectroscopy, light scattering, and disk centrifugation.

The compositions of the invention comprise metaxalone nanoparticles which have an effective average particle size of less than about 2000 nm (*i*.*e*., 2 microns), less than about 1900 nm, less than less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 140 nm, less than about 130 nm, less than about 120 nm, less than about 110 nm, less than about 100 nm, less than about 90 nm, less than about 80 nm, less than about 70 nm, less than about 60 nm, or less than about 50 nm, when measured by the above-noted techniques.

By "an effective average particle size of less than about 2000 nm" it is meant that at least 50% of the nanoparticulate metaxalone particles have a weight average particle size of less than about 2000 nm, when measured by the above-noted techniques. In other embodiments of the invention, at least about 70%, at least about 90%, at least about 95%, or at least about 99% of the nanoparticulate metaxalone particles have a particle size of less than the effective average, by weight, i.e., less than about 2000 nm, less than about 1900 nm, less than less than about 1800 nm, less than about 1700 nm, *etc*.

If the nanoparticulate metaxalone composition is combined with a microparticulate metaxalone or non-metaxalone active agent composition, then such a composition is either solubilized or has an effective average particle size of greater than about 2 microns. By "an effective average particle size of greater than about 2 microns" it is meant that at least 50% of the microparticulate metaxalone or non-metaxalone active agent particles have a particle size of greater than about 2 microns, by weight, when measured by the above-noted techniques. In other embodiments of the invention, at least about 70%, at least about 90%, at least about 95%, or at least about 99%, by weight, of the microparticulate metaxalone or non-metaxalone active agent particles have a particle size greater than about 2 microns.

In the present invention, the value for D50 of a nanoparticulate metaxalone composition is the particle size below which 50% of the metaxalone particles fall, by weight. Similarly, D90 and D99 are the particle sizes below which 90% and 99%, respectively, of the metaxalone particles fall, by weight.

### 5. Concentration of Nanoparticulate Metaxalone and Surface Stabilizers

The relative amounts of metaxalone and one or more surface stabilizers can vary widely. The optimal amount of the individual components can depend, for example, upon the hydrophilic lipophilic balance (HLB), melting point, and the surface tension of water solutions of the stabilizer, *etc*.

The concentration of metaxalone can vary from about 99.5% to about 0.001 %, from about 95% to about 0.1%, or from about 90% to about 0.5%, by weight, based on the total combined dry weight of the metaxalone and at least one surface stabilizer, not including other excipients.

The concentration of the at least one surface stabilizer can vary from about 0.5% to about 99.999%, from about 5.0% to about 99.9%, or from about 10% to about 99.5%, by weight, based on the total combined dry weight of the metaxalone and at least one surface stabilizer, not including other excipients.

### C. Methods of Making Nanoparticulate Metaxalone Formulations

The nanoparticulate metaxalone compositions can be made using, for example, milling, homogenization, or precipitation techniques. Exemplary methods of making nanoparticulate compositions are described in the '684 patent. Methods of making nanoparticulate compositions are also described in U.S. Patent No. 5,518,187 for "Method of Grinding Pharmaceutical Substances;" U.S. Patent No. 5,718,388 for "Continuous Method of Grinding Pharmaceutical Substances;" U.S. Patent No. 5,862,999 for "Method of Grinding Pharmaceutical Substances;" U.S. Patent No. 5,665,331 for "Co-Microprecipitation of Nanoparticulate Pharmaceutical Agents with Crystal Growth Modifiers;" U.S. Patent No. 5,662,883 for "Co-Microprecipitation of Nanoparticulate Pharmaceutical Agents with Crystal Growth Modifiers;" U.S. Patent No. 5,560,932 for "Microprecipitation of Nanoparticulate Pharmaceutical Agents;" U.S. Patent No. 5,543,133 for "Process of Preparing X-Ray Contrast Compositions Containing Nanoparticles;" U.S. Patent No. 5,534,270 for "Method of Preparing Stable Drug Nanoparticles;" U.S. Patent No. 5,510,118 for "Process of Preparing Therapeutic Compositions Containing Nanoparticle;" and U.S. Patent No. 5,470,583 for "Method of Preparing Nanoparticle Compositions Containing Charged Phospholipids to Reduce Aggregation".

Following milling, homogenization, precipitation, etc., the resultant nanoparticulate metaxalone composition can be utilized in solid or liquid dosage formulations, such as controlled release formulations, solid dose fast melt formulations, aerosol formulations, nasal formulations, lyophilized formulations, tablets, capsules, solid lozenge, powders, creams, ointments, *etc*.

### 1. Milling to Obtain Nanoparticulate Metaxalone Dispersions

Milling metaxalone to obtain a nanoparticulate dispersion comprises dispersing metaxalone particles in a liquid dispersion media in which metaxalone is poorly soluble, followed by applying mechanical means in the presence of grinding media to reduce the particle size of metaxalone to the desired effective average particle size. The dispersion media can be, for example, water, safflower oil, ethanol, t-butanol, glycerin, polyethylene glycol (PEG), hexane, or glycol.

The metaxalone particles can be reduced in size in the presence of at least one surface stabilizer. Alternatively, the metaxalone particles can be contacted with one or more surface stabilizers after attrition. Other compounds, such as a diluent, can be added to the metaxalone/surface stabilizer composition during the size reduction process. Dispersions can be manufactured continuously or in a batch mode.

### 2. Precipitation to Obtain Nanoparticulate Metaxalone Compositions

Another method of forming the desired nanoparticulate metaxalone composition is by microprecipitation. This is a method of preparing stable dispersions of poorly soluble active agents in the presence of one or more surface stabilizers and one or more colloid stability enhancing surface active agents free of any trace toxic solvents or solubilized heavy metal impurities. Such a method comprises, for example: (1) dissolving metaxalone in a suitable solvent; (2) adding the formulation from step (1) to a solution comprising at least one surface stabilizer; and (3) precipitating the formulation from step (2) using an appropriate non-solvent. The method can be followed by removal of any formed salt, if present, by dialysis or diafiltration and concentration of the dispersion by conventional means.

### 3. Homogenization to Obtain Metaxalone Nanoparticulate Compositions

Exemplary homogenization methods of preparing active agent nanoparticulate compositions are described in U.S. Patent No. 5,510,118, for "Process of Preparing Therapeutic Compositions Containing Nanoparticles."

Such a method comprises dispersing metaxalone particles in a liquid dispersion media in which metaxalone is poorly soluble, followed by subjecting the dispersion to homogenization to reduce the particle size of the metaxalone to the desired effective average particle size. The dispersion media can be, for example, water, safflower oil, ethanol, t-butanol, glycerin, polyethylene glycol (PEG), hexane, or glycol.

The metaxalone particles can be reduced in size in the presence of at least one surface stabilizer. Alternatively, the metaxalone particles can be contacted with one or more surface stabilizers either before or after attrition. Other compounds, such as a diluent, can be added to the metaxalone/surface stabilizer composition either before, during, or after the size reduction process. Dispersions can be manufactured continuously or in a batch mode.

### D. Methods of Using Nanoparticulate Metaxalone Formulations

The method of the invention comprises administering to a subject an effective amount of a composition comprising nanoparticulate metaxalone. The metaxalone compositions of the present invention can be administered to a subject via any conventional means including, but not limited to, orally, rectally, ocularly, parenterally (e.g., intravenous, intramuscular, or subcutaneous), intracisternally, pulmonary, intravaginally, intraperitoneally, locally (e.g., powders, ointments or drops), or as a buccal or nasal spray. As used herein, the term "subject" is used to mean an animal, preferably a mammal, including a human or non-human. The terms patient and subject may be used interchangeably.

The USFDA has approved indications for musculoskeletal relaxants, such as metaxalone, as adjuncts to rest and physical therapy for relief of acute, painful musculoskeletal problems. Clinically, the mild pain associated with the majority of cases of minor muscle strains and minor injuries is self limiting. Most patients usually respond rapidly to rest. An anti-inflammatory drug may be useful when there is considerable tissue damage and edema. In contrast, severe musculoskeletal strains and sprains, trauma, and cervical or lumbar radiculopathy as a consequence of degenerative osteoarthritis, herniated disk, spondylitis or laminectomy, often cause moderate or severe and more chronic painful skeletal muscle spasm. The principal symptoms include local pain, tenderness on palpation, increased muscle consistency and limitation of motion. For these patients skeletal muscle relaxants alone or in combination with an analgesic are frequently prescribed. Results of some studies have suggested that a formulation of a muscle relaxant and an analgesic provides greater benefit in patients with acute musculoskeletal problems than similar doses of an analgesic alone.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents, or vehicles including water, ethanol, polyols (propyleneglycol, polyethylene-glycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions, and by the use of surfactants.

The nanoparticulate compositions may also contain adjuvants such as preserving, wetting, emulsifying, and dispensing agents. Prevention of the growth of microorganisms can be ensured by various antibacterial and antifungal agents, such as parabens, chlorobutanol, phenol, sorbic acid, and the like. It may also be desirable to include isotonic agents, such as sugars, sodium chloride, and the like. Prolonged absorption of the injectable pharmaceutical form can be brought about by the use of agents delaying absorption, such as aluminum monostearate and gelatin.

Solid dosage forms for oral administration include, but are not limited to, powder aerosols, capsules, tablets, pills, powders, and granules. In such solid dosage forms, the active agent is admixed with at least one of the following: (a) one or more inert excipients (or carriers), such as sodium citrate or dicalcium phosphate; (b) fillers or extenders, such as starches, lactose, sucrose, glucose, mannitol, and silicic acid; (c) binders, such as carboxymethylcellulose, alignates, gelatin, polyvinylpyrrolidone, sucrose, and acacia; (d) humectants, such as glycerol; (e) disintegrating agents, such as agar-agar, calcium carbonate, potato or tapioca starch, alginic acid, certain complex silicates, and sodium carbonate; (f) solution retarders, such as paraffin; (g) absorption accelerators, such as quaternary ammonium compounds; (h) wetting agents, such as cetyl alcohol and glycerol monostearate; (i) adsorbents, such as kaolin and bentonite; and (j) lubricants, such as talc, calcium stearate, magnesium stearate, solid polyethylene glycols, sodium lauryl sulfate, or mixtures thereof. For capsules, tablets, and pills, the dosage forms may also comprise buffering agents.

Liquid dosage forms for oral administration include pharmaceutically acceptable aerosols, emulsions, solutions, suspensions, syrups, and elixirs. In addition to the active agent, the liquid dosage forms may comprise inert diluents commonly used in the art, such as water or other solvents, solubilizing agents, and emulsifiers. Exemplary emulsifiers are ethyl alcohol, isopropyl alcohol, ethyl carbonate, ethyl acetate, benzyl alcohol, benzyl benzoate, propyleneglycol, 1,3-butyleneglycol, dimethylformamide, oils, such as cottonseed oil, groundnut oil, corn germ oil, olive oil, castor oil, and sesame oil, glycerol, tetrahydrofurfuryl alcohol, polyethyleneglycols, fatty acid esters of sorbitan, or mixtures of these substances, and the like.

Besides such inert diluents, the composition can also include adjuvants, such as wetting agents, emulsifying and suspending agents, sweetening, flavoring, and perfuming agents.

One of ordinary skill will appreciate that effective amounts of metaxalone can be determined empirically and can be employed in pure form or, where such forms exist, in pharmaceutically acceptable salt, ester, or prodrug form. Actual dosage levels of metaxalone in the nanoparticulate compositions of the invention may be varied to obtain an amount of metaxalone that is effective to obtain a desired therapeutic response for a particular composition and method of administration. The selected dosage level therefore depends upon the desired therapeutic effect, the route of administration, the potency of the administered metaxalone, the desired duration of treatment, and other factors.

Dosage unit compositions may contain such amounts of such submultiples thereof as may be used to make up the daily dose. It will be understood, however, that the specific dose level for any particular patient will depend upon a variety of factors: the type and degree of the cellular or physiological response to be achieved; activity of the specific agent or composition employed; the specific agents or composition employed; the age, body weight, general health, sex, and diet of the patient; the time of administration, route of administration, and rate of excretion of the agent; the duration of the treatment; drugs used in combination or coincidental with the specific agent; and like factors well known in the medical arts.

### Example 1

The purpose of this example was to prepare a nanoparticulate dispersion of metaxalone.

A nanoparticulate colloidal dispersion (NCD) of metaxalone, comprising a mixture of 25% (w/w) metaxalone (UniChem Laboratories Ltd. (Germany)), 5% (w/w) polyvinylpyrolidine (PVP) (Plasdone^{®} K29/32; ISP Technologies), and 0.1 % docusate sodium (DOSS) (Cytec Industries, Inc.), was prepared by wet (aqueous) milling the mixture for 1.5 hours under high energy milling conditions in a DYNO®-Mill KDL (Willy A. Bachofen AG, Maschinenfabrik, Basel, Switzerland) equipped with a 150 cc batch chamber and utilizing 500 µm polymeric attrition media (Dow Chemical Co.).

The final mean (weight) metaxalone particle size was 334 nm, with a D50 of 319 nm, a D90 of 479 nm, and a D95 of 540 nm, as measured with a Horiba LA-910 particle size analyzer (Horiba Instruments, Irvine, CA).

### Example 2

The purpose of this example was to prepare a nanoparticulate dispersion of metaxalone.

A nanoparticulate colloidal dispersion (NCD) of metaxalone, comprising a mixture of 25% (w/w) metaxalone (UniChem Laboratories Ltd. (Germany)), 10% (w/w) PVP (Plasdone^{®} K29/32; ISP Technologies), and 0.25% DOSS (Cytec Industries, Inc.), was prepared by wet (aqueous) milling the mixture for 6.5 hours under high energy milling conditions in a DYNO®-Mill KDL (Willy A. Bachofen AG, Maschinenfabrik, Basel, Switzerland) equipped with a 300 cc re-circulation chamber and utilizing 500 µm polymeric attrition media (Dow Chemical Co.).

The final mean (weight) metaxalone particle size was 400 nm, with a D50 of 352 nm, a D90 of 628 nm, and a D95 of 768 nm, as measured with a Horiba LA-910 particle size analyzer (Horiba Instruments, Irvine, CA.

### Example 3

The purpose of this example was to prepare a nanoparticulate dispersion of metaxalone.

A nanoparticulate colloidal dispersion (NCD) of metaxalone, comprising a mixture of 35% (w/w) metaxalone (UniChem Laboratories Ltd. (Germany)), 7% (w/w) PVP (Plasdone^{®} K29/32; ISP Technologies), and 0.14% DOSS (Cytec Industries, Inc.), was prepared by wet (aqueous) milling the mixture for 8.4 hours under high energy milling conditions in a two liter Netzsch LMZ-2 Mill (Netzsch, Fxton. PA) equipped with a 20 liter re-circulation vessel and utilizing 500 µm polymeric attrition media (Dow Chemical Co.)

The final mean (weight) metaxalone particle size was 370 nm, with a D50 of 348 nm, a D90 of 344 nm, and a D95 of 624 nm, as measured with a Horiba LA-910 particle size analyzer (Horiba Instruments, Irvine, CA).

### Example 4

The purpose of this example was to prepare a nanoparticulate dispersion of metaxalone.

A nanoparticulate coiloidal dispersion (NCD) of metaxalone, comprising a mixture of 35% (w/w) metaxalone (UniChem Laboratories Ltd. (Germany)), 8.75% (w/w) PVP (Plasdone^{®} K29/32; ISP Technologies), and 0.35% DOSS (Cytec Industries, Inc.), was prepared by wet (aqueous) milling the mixture for 6.8 hours under high energy milling conditions in a two liter Netzsch LMZ-2 Mill (Netzsch, Exton, PA) equipped with a 20 liter re-circulation vessel and utilizing 500 µm polymeric attrition media (Dow Chemical Co.).

The final mean (weight) metaxalone particle size was 383 nm, with a D50 of 353 nm, a D90 of 572 nm, and a D95 of 668 nm, as measured with a Horiba LA-910 particle size analyzer (Horiba Instruments, Irvine, CA).

### Example 5

The purpose of this example was to determine the in vivo pharmacokinetics of nanoparticulate metaxalone compositions.

Three formulations were used in the study: two nanoparticulate metaxalone formulations and a microparticulate metaxalone formulation, Skelaxin^{®}.

### Formulation # 1: Dispersion of Nanoparticulate Metaxalone

20.0 g metaxalone (UniChem Laboratories Ltd. (Germany)) was added to a solution containing 4.0 g PVP (Plasdone^{®} K29/32; ISP Technologies), 0.08 g DOSS (Cytec Industries, Inc), and 56.0 g water, followed by milling of the resultant mixture for 180 minutes in a DYNO-Mill KDL (Willy A. Bachofen AG, Maschinenfabrik, Basel, Switzerland) at 2500 RPM.with 500 µm polymeric attrition media (Dow Chemical Co.).

The final mean (weight) metaxalone particle size was 381 nm, with a D50 of 330 nm, a D90 of 614 nm, and a D95 of 766 nm, as measured with a Horiba LA-910 particle size analyzer (Horiba Instruments, Irvine, CA). The formulation was diluted with water to 10% (w/w) metaxalone just prior to dosing.

### Formulation # 2: Dispersion of Nanoparticulate Metaxalone

20.0 g metaxalone (UniChem Laboratories Ltd. (Germany)) was added to a solution containing 6.4 g Lysozyme (Fordras, Lugano, Switzerland), and 53.6 g water, and milled for 80 minutes in a DYNO-Mill KDL (Willy A. Bachofen AG, Maschinenfabrik, Basel, Switzerland) at 4200 RPM with 500 µm polymeric attrition media (Dow Chemical Co.).

The final mean (weight) metaxalone particle size was 139 nm, with a D50 of 134 nm, a D90 of 190 nm, and a D95 of 210 nm, as measured with a Horiba LA-910 particle size analyzer (Horiba Instruments, Irvine, CA). The formulation was diluted with water to 10% (w/w) metaxalone just prior to dosing.

### Formulation # 3: Skelaxin^{®} tablet

The third formulation consisted of a 100 mg dose of a 400 mg Skelaxin^{®} tablet (Elan Pharmaceuticals, Inc.) (*i*.*e*., one quarter of the tablet).

| **TABLE 1** | | | |
|---|---|---|---|
| **Summary of Formulations Used in the Dog Study** | | | |
| **Formulation** | **Key Ingredients** | **Metaxalone Particle Size** | **Dosage Form & Quantity** |
| #1 | metaxalone, PVP K29/32, DOSS, and water | 381 nm | dispersion, 100 mg |
| #2 | Metaxalone, lysozyme, and water | 139 nm | dispersion, 100 mg |
| #3 | Skelaxin^{®} (metaxalone) tablet | microparticulate | tablet, 100 mg |

### Dog Study Protocol

Twelve male Beagle dogs were used in a fed/fasted metaxalone study. The studied dose was 100 mg. The twelve dogs were divided into three groups. Each group received one only one of the three formulations summarized in Table 1, below, under both fed and fasted conditions.

| **TABLE 2: Study Design** | | | |
|---|---|---|---|
| **Dog No.** | **Formulation** | **Administration** | **Comment** |
| 1,2,3,4 | Nanoparticulate Metaxalone Dispersion #1 | Oral gavage | Fed and fasted |
| 5,7,8,9 | Nanoparticulate Metaxalone Dispersion #2 | Oral gavage | Fed and fasted |
| 11,12,13,14 | One quarter of Skelaxin^{®} tablet | Orally | Fed and fasted |

**Fasted Conditions:** The animals were fasted (food only) for 12 to 16 hours prior to dosing on Day 1. On Day 1, 8 dogs were administered Formulations #1 or #2 by oral gavage. Following dosing, the gavage tube was flushed with 18 mL of water. The actual weight of each dose was gravimetrically determined by weighing the full dosing syringe, prior to and the empty syringe immediately following dose administration. The third formulation was administered orally as a ¼ tablet to 4 dogs. The weight of the tablet was recorded prior to dosing. Ten mL of water was given after dosing.
**Fed Conditions:** On Day 7 the dogs were fed with canned food mixed with the diet. The amount consumed was recorded as 0, 25, 50, 75 or 100%. Eight dogs were dosed with Formulation #1 or #2 by gavage approximately 10-30 minutes after eating. Following dosing, the gavage tube was flushed with 18 mL of water. The actual weight of each dose was gravimetrically determined by weighing the full dosing syringe, prior to and the empty syringe immediately following dose administration. Four dogs were dosed with a ¼ tablet given orally. The weight of the tablet was recorded prior to dosing. Ten mL of was given after dosing.

Table 3 below provides a summary of the study protocol.

| **TABLE 3** | | | | | |
|---|---|---|---|---|---|
| **Study Protocol** | | | | | |
| **Treatment period** | **Fed vs. fasted** | ***n*** | **Route of administration** | **Treatment** | **Dose** |
| Week 1 | Fasted | 4 | PO | Formulation #1 (10% metaxalone) | 100 mg |
| | | 4 | PO | Formulation #2 (10% metaxalone) | 100 mg |
| | | 4 | PO | ¼ Skelaxin® tablet | 100 mg |
| Week 2 | Fed | 4 | PO | Formulation #1 (10% metaxalone) | 100 mg |
| | | 4 | PO | Formulation #2 (10% metaxalone) | 100 mg |
| | | 4 | PO | ¼ Skelaxin® tablet | 100 mg |

**Collection of Blood Samples:** Blood samples were collected predose, at 10, 20, 30, 45, and 60 minutes, and at 1.5, 2, 4, 8, 12, and 24 hours after oral doses. Approximately three mL of whole blood was collected from the jugular vein into Vacutainer tubes containing sodium heparin anticoagulant and placed on ice until placed into a refrigerated centrifuge for preparation of plasma. Following centrifugation, the plasma was placed on ice, and dispensed into approximately duplicate aliquots. One aliquot was frozen at approximately -70°C.

Cₘₐₓ, Tₘₐₓ, and AUC data were calculated from an analysis of the blood samples. The data is shown below in Table 4.

| **Table 4** | | | |
|---|---|---|---|
| **Pharmacokinetic Data** | | | |
| **Formulation** | **Cₘₐₓ (ng/mL)** | **Tₘₐₓ (hours)** | **AUC (ng hr/ml)** |
| #1, Fasted | 2582.7 | 0.60 | 2364.1 |
| #1, Fed | 2928.5 | 0.40 | 1373.8 |
| #2, Fasted | 3454.5 | 0.37 | 3087.9 |
| #2, Fed | 1045.0 | 0.38 | 931.2 |
| Skelaxin^{®}, Fasted | 174.9 | 1.19 | 246.5 |
| Skelaxin^{®}, Fed | 343.7 | 2.19 | 457.0 |

The average metaxalone plasma concentration over time (0-6 hours) for Formulation #1, #2, and the ¼ Skelaxin^{®} tablet is shown in Figure 1 (fasted conditions) and Figure 2 (fed conditions).

The data provided in Table 4 and Figures 1 and 2 show that the nanoparticulate metaxalone compositions had dramatically greater Cₘₐₓ and AUC, and dramatically lower Tₘₐₓ. Thus, the nanoparticulate metaxalone formulations showed significantly greater bioavailability, and a significantly faster onset of action, under *both* fed and fasted conditions, as compared to the microparticulate formulation of metaxalone, Skelaxin^{®}.

For example, under fasted conditions, Formulations #1 and #2 had a Cₘₐₓ of 2582.7 ng/mL and 3454.5 ng/mL, respectively, in contrast to the Cₘₐₓ of 174.9 ng/mL for Skelaxin^{®}. This translates into an increase in Cₘₐₓ of about 1392% for Formulation #1, and about 1875% for Formulation #2, over the microparticulate formulation of metaxalone, Skelaxin^{®}.

Under fed conditions, Formulations #1 and #2 had a Cₘₐₓ of 2928.5 ng/mL and 1045.0 ng/mL, respectively, in contrast to the Cₘₐₓ of 343.7 ng/mL for Skelaxin^{®}. This translates into an increase in Cₘₐₓ of about 752% for Formulation #1, and about 204% for Formulation #2, over the microparticulate formulation of metaxalone, Skelaxin^{®}.

Similarly, under fasted conditions, Formulations #1 and #2 had an AUC of 2364.1 ng/mL and 3087.9 ng/mL, respectively, in contrast to the AUC of 246.5 ng/mL for Skelaxin^{®}. This translates into an increase in AUC of about 859% for Formulation #1, and about 1153% for Formulation #2, over the microparticulate formulation of metaxalone, Skelaxin^{®}.

Under fed conditions, Formulations #1 and #2 had an AUC of 1373.8 ng/mL and 931.2 ng/mL, respectively, in contrast to the AUC of 457.0 ng/mL for Skelaxin^{®}. This translates into an increase in AUC of about 201% for Formulation #1, and about 104% for Formulation #2, over the microparticulate formulation of metaxalone, Skelaxin^{®}.

The Tₘₐₓ results were equally surprising. Under fasted conditions, Formulations #1 and #2 had a Tₘₐₓ of 0.60 hours and 0.37 hours, respectively, in contrast to the Tₘₐₓ of 1.19 hours for Skelaxin^{®}. This translates into a decrease in Tₘₐₓ of about 50% for Formulation #1, and about 69% for Formulation #2, over the microparticulate formulation of metaxalone, Skelaxin^{®}.

Similarly, under fed conditions, Formulations #1 and #2 had a Tₘₐₓ of 0.40 hours and 0.38 hours, respectively, in contrast to the Tₘₐₓ of 2.19 hours for Skelaxin^{®}. This translates into a decrease in Tₘₐₓ of about 82% for Formulation #1, and about 83% for Formulation #2, over the microparticulate formulation of metaxalone, Skelaxin^{®}.

Although the sample size is very small, the data in this example suggests the two nanoparticulate metaxalone formulations (Formulation #1 and #2) are more bioavailable than the commercial Skelaxin^{®} formulation.

### Example 6

The purpose of this example was to prepare a nanoparticulate metaxalone composition utilizing the surface stabilizers Plasdone® S630, which is a random copolymer of vinyl acetate and vinyl pyrrolidone, and DOSS.

A mixture of 5% metaxalone, 2% Plasdone® S630, and 0.1% DOSS was milled in an aqueous environment in a DynoMill® (Type: KDL; Mfg.: Willy Bachofen, Basel, Switzerland) for 4 hours, until the mean particle size of the metaxalone particles was 513 nm, with a D90 of 790 nm. After 1 day at 5°C, the composition had a mean metaxalone particle size of 492 nm and a D90 of 730 nm. After 1 week at 5°C, the composition had a mean metaxalone particle size of 548 nm and a D90 of 864 nm. After 2 weeks at 5°C, the composition had a mean metaxalone particle size of 392 nm and a D90 of 609 nm.
Particle size was measured using a with a Horiba LA-910 particle size analyzer (Horiba Instruments, Irvine, CA).

### Example 7

The purpose of this example was to prepare a nanoparticulate metaxalone composition utilizing the surface stabilizers Plasdone® S630 and DOSS.

A mixture of 5% metaxalone, 1% Plasdone® S630, and 0.05% DOSS was milled in an aqueous environment in a DynoMill® (Type: KDL; Mfg.: Willy Bachofen, Basel, Switzerland) for 2.5 hours, until the mean particle size of the metaxalone particles was 655 nm, with a D90 of 1217 nm. Particle size was measured using a with a Horiba LA-910 particle size analyzer (Horiba Instruments, Irvine, CA).

### Example 8

The purpose of this example was to prepare a nanoparticulate metaxalone composition utilizing the surface stabilizers hydroxypropylmethyl cellulose (HPMC) and DOSS.

A mixture of 5% metaxalone, 2% HPMC, and 0.1% DOSS was milled in an aqueous environment in a DynoMill® (Type: KDL; Mfg.: Willy Bachofen, Basel, Switzerland) for 3 hours, until the mean particle size of the metaxalone particles was 596 nm, with a D90 of 1171 nm. After 1 day at 5°C, the composition had a mean metaxalone particle size of 608 nm and a D90 of 1274 nm. After 1 week at 5°C, the composition had a mean metaxalone particle size of 584 nm and a D90 of 1107 nm. After 2 weeks at 5°C, the composition had a mean metaxalone particle size of 556 nm and a D90 of 990 nm. Particle size was measured using a with a Horiba LA-910 particle size analyzer (Horiba Instruments, Irvine, CA).

### Example 9

The purpose of this example was to prepare a nanoparticulate metaxalone composition utilizing the surface stabilizers hydroxypropyl cellulose (HPC-SL). A mixture of 5% metaxalone and 2% HPC-SL was milled in an aqueous environment in a DynoMill® (Type: KDL; Mfg.: Willy Bachofen, Basel, Switzerland) for 3 hours, until the mean particle size of the metaxalone particles was 317 nm, with a D90 of 454 nm. After 1 day at 5°C, the composition had a mean metaxalone particle size of 336 nm and a D90 of 489 nm; after 1 day at 25°C, the composition had a mean metaxalone particle size of 372 nm; and after 1 day at 40°C, the composition had a mean metaxalone particle size of 433 nm. After 1 week at 5°C, the composition had a mean metaxalone particle size of 349 nm and a D90 of 509 nm; after 1 week at 25°C, the composition had a mean metaxalone particle size of 479 nm; and after 1 week at 40°C, the composition had a mean metaxalone particle size of 617 nm. Particle size was measured using a with a Horiba LA-910 particle size analyzer (Horiba Instruments, Irvine, CA).

### Example 10

The purpose of this example was to prepare a nanoparticulate metaxalone composition utilizing the surface stabilizers polyvinylpyrrolidone (PVP) and DOSS.

A mixture of 5% metaxalone, 2% PVP, and 0.05% DOSS was milled in an aqueous environment in a DynoMill® (Type: KDL; Mfg.: Willy Bachofen, Basel, Switzerland) for 3 hours, until the mean particle size of the metaxalone particles was 363 nm, with a D90 of 550 nm. After 1 day at 5°C, the composition had a mean metaxalone particle size of 363 nm and a D90 of 529 nm; after 1 day at 25°C, the composition had a mean metaxalone particle size of 440 nm; and after 1 day at 40°C, the composition had a mean metaxalone particle size of 761 nm. After 13 days at 5°C, the composition had a mean metaxalone particle size of 386 nm and a D90 of 569 nm; after 13 days at 25°C, the composition had a mean metaxalone particle size of 581 nm; and after 13 days at 40°C, the composition had a mean metaxalone particle size of 518 nm. Particle size was measured using a with a Horiba LA-910 particle size analyzer (Horiba Instruments, Irvine, CA).

### Example 11

The purpose of this example was to prepare a nanoparticulate metaxalone composition utilizing the surface stabilizers PVP and DOSS.

A mixture of 20% metaxalone, 8% PVP, and 0.2% DOSS was milled in an aqueous environment in a DynoMill® (Type: KDL; Mfg.: Willy Bachofen, Basel, Switzerland) for 4 hours, until the mean particle size of the metaxalone particles was 386 nm. After 10 days at 5°C, the composition had a mean metaxalone particle size of 415 nm; and after 10 days at 25°C, the composition had a mean metaxalone particle size of 515 nm. After 24 days at 5°C, the composition had a mean metaxalone particle size of 420 nm; and after 24 days at 25°C, the composition had a mean metaxalone particle size of 548 nm. Particle size was measured using a with a Horiba LA-910 particle size analyzer (Horiba Instruments, Irvine, CA).

### Example 12

The purpose of this example was to prepare a nanoparticulate metaxalone composition utilizing the surface stabilizers Plasdone® S630 and DOSS.

A mixture of 20% metaxalone, 8% Plasdone® S630, and 0.1% DOSS was milled in an aqueous environment in a DynoMill® (Type: KDL; Mfg.: Willy Bachofen, Basel, Switzerland) for 1.5 hours, until the mean particle size of the metaxalone particles was 408 nm. After 4 days at 5°C, the composition had a mean metaxalone particle size of 435 nm; and after 4 days at 25°C, the composition had a mean metaxalone particle size of 508 nm. After 18 days at 5°C, the composition had a mean metaxalone particle size of 440 nm; and after 18 days at 25°C, the composition had a mean metaxalone particle size of 533 nm. Particle size was measured using a with a Horiba LA-910 particle size analyzer , (Horiba Instruments, Irvine, CA).

### Example 13

The purpose of this example was to prepare a nanoparticulate metaxalone composition utilizing the surface stabilizers PVP and DOSS.

A mixture of 20% metaxalone, 2% Plasdone® S630, and 0.2% DOSS was milled in an aqueous environment in a DynoMill® (Type: KDL; Mfg.: Willy Bachofen, Basel, Switzerland) for 1.5 hours, until the mean particle size of the metaxalone particles was 425nm. After 4 days at 5°C, the composition had a mean metaxalone particle size of 407 nm; and after 4 days at 25°C, the composition had a mean metaxalone particle size of 550 nm. After 18 days at 5°C, the composition had a mean metaxalone particle size of 419 nm; and after 18 days at 25°C, the composition had a mean metaxalone particle size of 740 nm. Particle size was measured using a with a Horiba LA-910 particle size analyzer (Horiba Instruments, Irvine, CA).

### Example 14

The purpose of this example was to prepare a nanoparticulate metaxalone composition utilizing the surface stabilizer HPC-SL.

A mixture of 10% metaxalone and 4% HPC-SL was milled in an aqueous environment in a DynoMill® (Type: KDL; Mfg.: Willy Bachofen, Basel, Switzerland) for 180 minutes, until the mean particle size of the metaxalone particles was 358 nm. After 13 days at 5°C, the composition had a mean metaxalone particle size of 410 nm; after 3 days at 25°C, the composition had a mean metaxalone particle size of 448 nm; and after 3 days at 40°C, the composition had a mean metaxalone particle size of 739 nm. Particle size was measured using a with a Horiba LA-910 particle size analyzer (Horiba Instruments, Irvine, CA).

## Claims

1. A composition comprising:
(a) particles of metaxalone or a salt thereof, wherein the metaxalone particles have an effective average particle size of less than 2000 nm; and
(b) at least one surface stabilizer, in which the surface stabilizer is adsorbed on the surface of the metaxalone particles,
with the proviso that the composition does not consist of d-alpha tocopherol succinate 200 mg; Cremophor RH 40 200 mg; glycerol dibehenate (Compritol 888) 100mg; glycenol distearate (Precivol) 80mg; and metaxalone 300 mg·

2. A composition according to claim 1, wherein the metaxalone is selected from the group consisting of a crystalline phase, an amorphous phase, a semi-crystalline phase, a semi-amorphous phase, and mixtures thereof

3. A composition according to claim 1 or claim 2, wherein the effective average particle size of the metaxalone particles is selected from the group consisting of less than µ 1900 nm, less than 1800 nm, less than 1700 nm, less than 1600 nm, less than 1500 nm, less than 1400 nm, less than 1300 nm, less than 1200 nm, less than 1100 nm, less than 1000 nm, less than 900 nm, less than 800 nm, less than 700 nm, less than 600 nm, less than 500 nm, less than 400 nm, less than 300 nm, less than 250 nm, less than 200 nm, less than 100 nm, less than 75 nm, and less than 50 nm.

4. A composition according to any one of claims 1 to 3, wherein the composition is formulated:
(a) for administration selected from the group consisting of oral, pulmonary, rectal, ophthalmic, colonic, parenteral, intracisternal, intravaginal, intraperitoneal, local, buccal, nasal, and topical administration; and/or
(b) into a dosage form selected from the group consisting of liquid dispersions, oral suspensions, gels, aerosols, ointments, creams, controlled release formulations, fast melt formulations, lyophilized formulations, tablets, capsules, delayed release formulations, extended release formulations, pulsatile release formulations, and mixed immediate release and controlled release formulations.

5. A composition according to any one of claims 1 to 4, wherein:
(a) the metaxalone or a salt thereof is present in an amount selected from the group consisting of from 99.5% to 0.001%, from 95% to 0.1%, and from 90% to 0.5%, by weight, based on the total combined weight of the metaxalone or a salt thereof and at least one surface stabilizer, not including other excipients; and
(b) the at least one surface stabilizer is present in an amount selected from the group consisting of from 0.5% to 99.999% by weight, from 5.0% to 99.9% by weight, and from 10% to 99.5% by weight, based on the total combined dry weight of the metaxalone or a salt thereof and at least one surface stabilizer, not including other excipients.

6. A composition according to any one of claims 1 to 5, wherein at least one surface stabilizer:
(a) is selected from the group consisting of a nonionic surface stabilizer, an anionic surface stabilizer, a cationic surface stabilizer, a zwitterionic surface stabilizer, and an ionic surface stabilizer; and/or
(b) is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hypromellose, carboxymethylcellulose sodium, methylcellulose, hydroxyethylcellulose, hypromellose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers; poloxamines, a charged phospholipid, dioctylsulfosuccinate, dialkylesters of sodium sulfosuccinic acid, sodium lauryl sulfate, alkyl aryl polyether sulfonates, mixtures of sucrose stearate and sucrose distearate, p-isononylphenoxypoly-(glycidol), decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; lysozyme, PEG-phospholipid, PEG-cholesterol, PEG-cholesterol derivative, PEG-vitamin A, random copolymers of vinyl acetate and vinyl pyrrolidone, cationic polymers, cationic biopolymers, cationic polysaccharides, cationic cellulosics, cationic alginates, cationic nonpolymeric compounds, cationic phospholipids, cationic lipids, polymethylmethacrylate trimethylammonium bromide, sulfonium compounds, polyvinylpyrrolidone-2-dimethylaminoethyl methacrylate dimethyl sulfate, hexadecyltrimethyl ammonium bromide, phosphonium compounds, quaternary ammonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium chloride bromide, C₁₂₋₁₅dimethyl hydroxyethyl ammonium chloride, C₁₂₋₁₅dimethyl hydroxyethyl ammonium chloride bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkylbenzenealkyl ammonium chloride, lauryl trimethyl ammonium chloride, alkylbenzyl methyl ammonium chloride, alkyl benzyl dimethyl ammonium bromide, C₁₂ trimethyl ammonium bromides, C₁₅ trimethylammonium bromides, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT 10^{™}, tetrabutylammonium bromide, benzyl trimethylammonium bromide, choline esters, benzalkonium chloride, stearalkonium chloride compounds, cetyl pyridinium bromide, cetyl pyridinium chloride, halide salts of quatemized polyoxyethylalkylamines, MIRAPOL^{™}, ALKAQUAT^{™}, alkyl pyridinium salts; amines, amine salts, amine oxides, imide azolinium salts, protonated quaternary acrylamides, methylated quaternary polymers, and cationic guar.

7. A composition according to claim 6, wherein the composition is bioadhesive.

8. A composition according to any one of claims 1 to 7, comprising at least one primary surface stabilizer and at least one secondary surface stabilizer.

9. A composition according to any one of claims 1 to 8, comprising as a surface stabilizer polyvinylpyrrolidone, docusate sodium, lysozyme, or a combination thereof.

10. A composition according to any one of claims 1 to 9, further comprising at least one additional metaxalone composition having an effective average particle size which is different that the effective average particle size of the metaxalone composition of claim 1.

11. A composition according to any one of claims 1 to 10, additionally comprising one or more non-metaxalone active agents.

12. A composition according to claim 11, wherein:
(a) said additional one or more non-metaxalone active agents are selected from the group consisting of amino acids, proteins, peptides, nucleotides, anti-obesity drugs, central nervous system stimulants, carotenoids, corticosteroids, elastase inhibitors, anti-fungals, oncology therapies, anti-emetics, analgesics, cardiovascular agents, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives, astringents, alpha-adrenergic receptor blocking agents, beta-adrenoceptor blocking agents, blood products, blood substitutes, cardiac inotropic agents, contrast media, corticosteroids, cough suppressants, diagnostic agents, diagnostic imaging agents, diuretics, dopaminergics, haemostatics, immunological agents, lipid regulating agents, muscle relaxants, parasympathomimetics, parathyroid calcitonin, parathyroid biphosphonates, prostaglandins, radio-pharmaceuticals, sex hormones, anti-allergic agents, stimulants, anoretics, sympathomimetics, thyroid agents, vasodilators, xanthines, acyclovir, alprazolam, altretamine, amiloride, amiodarone, benztropine mesylate, bupropion, cabergoline, candesartan, cerivastatin, chlorpromazine, ciprofloxacin, cisapride, clarithromycin, clonidine, clopidogrel, cyclobenzaprine, cyproheptadine, delavirdine, desmopressin, diltiazem, dipyridamole, dolasetron, enalapril maleate, enalaprilat, famotidine, felodipine, furazolidone, glipizide, irbesartan, ketoconazole, lansoprazole, loratadine, loxapine, mebendazole, mercaptopurine, milrinone lactate, minocycline, mitoxantrone, nelfinavir mesylate, nimodipine, norfloxacin, olanzapine, omeprazole, penciclovir, pimozide, tacolimus, quazepam, raloxifenc, rifabutin, rifampin, risperidone, rizatriptan, saquinavir, sertraline, sildenafil, acetyl-sulfisoxazole, temazepam, thiabendazole, thioguanine, trandolapril, triamterene, trimetrexate, troglitazone, trovafloxacin, verapamil, vinblastine sulfate, mycophenolate, atovaquone, atovaquone, proguanil, ceftazidime, cefuroxime, etoposide, terbinafine, thalidomide, fluconazole, amsacrine, dacarbazine, teniposide, acetylsalicylate, an NSAID, and a COX-2 inhibitor; and/or
(b) said additional one or more non-metaxalone active agents is an NSAID selected from the group consisting of nabumetone, tiaramide, proquazone, bufexamac, flumizole, epirazole, tinoridine, timegadine, dapsone, aspirin, diflunisal, benorylate, fosfosal, diclofenac, alclofenac, fenclofenac, etodolac, indomethacin, sulindac, tolmetin, fentiazac, tilomisole, carprofen, fenbufen, flurbiprofen, ketoprofen, oxaprozin, suprofen, tiaprofenic acid, ibuprofen, naproxen, fenoprofen, indoprofen, pirprofen, flufenamic, mefenamic, meclofenamic, niflumic, oxyphenbutazone, phenylbutazone, apazone, feprazone, piroxicam, sudoxicam, isoxicam, and tenoxicam; and/or
(c) said additional one or more non-metaxalone active agents is a COX-2 inhibitor selected from the group consisting of celecoxib, rofecoxib, meloxicam, valdecoxib, parecoxib, etoricoxib, SC-236, NS-398, SC-58125, SC-57666, SC-558, SC-560, etodolac, DFU, monteleukast, L-745337, L-761066, L-761000, L-748780, DUP-697, PGV 20229, iguratimod, BF 389, PD 136005, PD 142893, PD 145065, PD 138387, flurbiprofen, nimesulide, nabumetone, flosulide, piroxicam, diclofenac, lumiracoxib, D 1367, diflumidone, JTE-522, FK-3311, FK 867, FR 115068, GR 253035, RWJ 63556, RWJ 20485, ZK 38997, S 2474, CL 1004, RS 57067, RS 104897 RS 104894, SC 41930, pranlukast, and SB 209670, heptinylsulfide, and FR 140423.

13. A composition according to any one of claims 1 to 12, wherein:
(a) upon administration to a mammal the metaxalone particles redisperse such that the particles have an effective average particle size selected from the group consisting of less than about 2 microns, less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, and less than about 50 nm; and/or
(b) the composition redisperses in a biorelevant media such that the metaxalone particles have an effective average particle size selected from the group consisting of less than about 2 microns, less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, and less than about 50 nm.

14. A composition according to claim 13, wherein the biorelevant media is selected from the group consisting of water, aqueous electrolyte solutions, aqueous solutions of a salt, aqueous solutions of an acid, aqueous solutions of a base, and combinations thereof.

15. A composition according to any one of claims 1 to 14, wherein:
(a) the Tₘₐₓ of the metaxalone, when assayed in the plasma of a mammalian subject following administration, is less than the Tₘₐₓ for a non-nanoparticulate metaxalone formulation, administered at the same dosage; and/or
(b) the Tₘₐₓ is selected from the group consisting of not greater than about 90%, not greater than about 80%, not greater than about 70%, not greater than about 60%, not greater than about 50%, not greater than about 30%, not greater than about 25%, not greater than about 20%, not greater than about 15%, not greater than about 10%, and not greater than about 5% of the Tₘₐₓ exhibited by a non-nanoparticulate metaxalone formulation, administered at the same dosage.

16. A composition according to any one of claims 1 to 15, wherein:
(a) the Cₘₐₓ of the metaxalone, when assayed in the plasma of a mammalian subject following administration, is greater than the Cₘₐₓ for a non-nanoparticulate metaxalone formulation, administered at the same dosage; and/or
(b) the Cₘₐₓ is selected from the group consisting of at least about 50%, at least about 100%, at least about 200%, at least about 300%, at least about 400%, at least about 500%, at least about 600%, at least about 700%, at least about 800%, at least about 900%, at least about 1000%, at least about 1100%, at least about 1200%, at least about 1300%, at least about 1400%, at least about 1500%, at least about 1600%, at least about 1700%, at least about 1800%, or at least about 1900% greater than the Cₘₐₓ exhibited by a non-nanoparticulate formulation of metaxalone, administered at the same dosage.

17. A composition according to any one of claims 1 to 16, wherein:
(a) the AUC of the metaxalone, when assayed in the plasma of a mammalian subject following administration, is greater than the AUC for a non-nanoparticulate metaxalone formulation, administered at the same dosage; and/or
(b) the AUC is selected from the group consisting of at least about 25%, at least about 50%, at least about 75%, at least about 100%, at least about 125%, at least about 150%, at least about 175%, at least about 200%, at least about 225%, at least about 250%, at least about 275%, at least about 300%, at least about 350%, at least about 400%, at least about 450%, at least about 500%, at least about 550%, at least about 600%, at least about 750%, at least about 700%, at least about 750%, at least about 800%, at least about 850%, at least about 900%, at least about 950%, at least about 1000%, at least about 1050%, at least about 1100%, at least about 1150%, or at least about 1200% greater than the AUC exhibited by the non-nanoparticulate formulation of metaxalone, administered at the same dosage.

18. A composition according to any one of claims 1 to 17 which does not produce significantly different absorption levels when administered under fed as compared to fasting conditions.

19. A composition according to claim 18, wherein the difference in absorption of the metaxalone composition of the invention, when administered in the fed versus the fasted state, is selected from the group consisting of less than about 100%, less than about 90%, less than about 80%, less than about 70%, less than about 60%, less than about 50%, less than about 40%, less than about 30%, less than about 25%, less than about 20%, less than about 15%, less than about 10%, less than about 5%, and less than about 3%.

20. A composition according to any one of claims 1 to 19, wherein administration of the composition to a human in a fasted state is bioequivalent to administration of the composition to a subject in a fed state.

21. A composition according to claim 20, wherein "bioequivalency" is established by:
(a) a 90% Confidence Interval of between 0.80 and 1.25 for both Cₘₐₓ and AUC, or
(b) a 90% Confidence Interval of between 0.80 and 1.25 for AUC and a 90% Confidence Interval of between 0.70 to 1.43 for Cₘₐₓ.

22. A composition according to any one of claims 1 to 21, wherein the composition further comprises one or more pharmaceutically acceptable excipients, carriers, or a combination thereof

23. Use of a composition according to any one of claims 1 to 22 for the preparation of a medicament.

24. A use according to claim 23, wherein the medicament is useful in treating indications selected from the group consisting of indications where musculoskeletal relaxants are typically used, degenerative osteoarthritis, herniated disk, spondylitis, laminectomy, severe musculoskeletal strains, severe musculoskeletal sprains, musculoskeletal trauma, cervical radiculopathy, lumbar radiculopathy, and a combination thereof.

25. A method of making a metaxalone composition according to anyone of claims 1 to 22 comprising contacting particles of metaxalone or a salt thereof with at least one surface stabilizer for a time and under conditions sufficient to provide a metaxalone composition having an effective average particle size of less than about 2000 nm.

26. A method according to claim 25, wherein said contacting comprises grinding, wet grinding, homogenizing, or precipitation.

## Patentansprüche

1. Zusammensetzung, umfassend:
(a) Metaxalon-Partikel oder ein Salz davon, wobei die Metaxalon-Partikel eine effektive durchschnittliche Partikelgröße von weniger als 2000 nm haben; und
(b) mindestens einen Oberflächenstabilisator, wobei der Oberflächenstabilisator an der Oberfläche der Metaxalon-Partikel adsorbiert wird, mit der Maßgabe, dass die Zusammensetzung nicht aus D-alpha-Tocopherolsuccinat 200 mg; Cremophor RH 40 200mg; Glyceroldibehenat (Compritol 888) 100 mg; Glyceroldistearat (Precirol) 80 mg und Metaxalon 300 mg besteht.

2. Zusammensetzung nach Anspruch 1, wobei das Metaxalon ausgewählt ist aus der Gruppe bestehend aus einer kristallinen Phase, einer amorphen Phase, einer semikristallinen Phase, einer semiamorphen Phase und Gemischen davon.

3. Zusammensetzung nach Anspruch 1 oder Anspruch 2, wobei die effektive durchschnittliche Partikelgröße der Metaxalon-Partikel ausgewählt ist aus der Gruppe bestehend aus weniger als 1900 nm, weniger als 1800 nm, weniger als 1700 nm, weniger als 1600 nm, weniger als 1500 nm, weniger als 1400 nm, weniger als 1300 nm, weniger als 1200 nm, weniger als 1100 nm, weniger als 1000 nm, weniger als 900 nm, weniger als 800 nm, weniger als 700 nm, weniger als 600 nm, weniger als 500 nm, weniger als 400 nm, weniger als 300 nm, weniger als 250 nm, weniger als 200 nm, weniger als 100 nm, weniger als 75 nm und weniger als 50 nm.

4. Zusammensetzung nach einem der Ansprüche 1 bis 3, wobei die Zusammensetzung formuliert ist:
(a) zur Verabreichung ausgewählt aus der Gruppe bestehend aus oraler, pulmonaler, rektaler, ophthalmischer, parenteraler, intrazisternaler, intravaginaler, intraperitonealer, örtlicher, bukkaler, nasaler, topischer Verabreichung und Verabreichung über den Dickdarm; und/oder
(b) in einer Darreichungsform ausgewählt aus der Gruppe bestehend aus flüssigen Dispersionen, oralen Suspensionen, Gelen, Aerosolen, Salben, Cremes, Formulierung mit kontrollierter Freisetzung, schnell zergehenden Formulierungen, Iyophilisierten Formulierungen, Tabletten, Kapseln, Formulierungen mit verzögerter Freisetzung, Formulierungen mit verlängerter Freisetzung, Formulierungen mit pulsierender Freisetzung und gemischten Formulierungen mit sofortiger und kontrollierter Freisetzung.

5. Zusammensetzung nach einem der Ansprüche 1 bis 4, wobei:
(a) das Metaxalon oder ein Salz davon in einer Menge vorliegt ausgewählt aus der Gruppe bestehend aus von 99,5 Gew.-% bis 0,001 Gew.-%, von 95 Gew.-% bis 0,1 Gew.-%, und von 90 Gew.-% bis 0,5 Gew.-%, basierend auf dem kombinierten Gesamtgewicht des Metaxalons oder eines Salzes davon und mindestens eines Oberflächenstabilisators, ohne weitere Arzneiträger; und
(b) der mindestens eine Oberflächenstabilisator in einer Menge vorliegt ausgewählt aus der Gruppe bestehend aus von 0,5 Gew.-% bis 99,999 Gew.-%, von 5 Gew.-% bis 99,9 Gew.-%, und von 10 Gew.-% bis 99,5 Gew.-%, basierend auf dem kombinierten Gesamttrockengewicht des Metaxalons oder eines Salzes davon und mindestens eines Oberflächenstabilisators, ohne weitere Arzneiträger.

6. Zusammensetzung nach einem der Ansprüche 1 bis 5, wobei der mindestens eine Oberflächenstabilisator:
(a) ausgewählt ist aus der Gruppe bestehend aus einem nicht-ionischen Oberflächenstabilisator, einem anionischen Oberflächenstabilisator, einem kationischen Oberflächenstabilisator, einem zwitterionischen Oberflächenstabilisator und einem ionischen Oberflächenstabilisator; und/oder
(b) ausgewählt ist aus der Gruppe bestehend aus Cetylpyridiniumchlorid, Gelatine, Casein, Phosphatiden, Dextran, Glycerin, Gummi arabicum, Cholesterin, Tragacanth, Stearinsäure, Benzalkoniumchlorid, Kalziumstearat, Glycerinmonostearat, Cetostearylalkohol, emulgierendes Cetomacrogol-Wachs, Sorbitanestern, Polyoxyethylenalkylethern, Polyoxyethylenrizinusöl-Derivaten, Polyoxyethylensorbitanfettsäureestern, Polyethylenglycolen, Dodecyltrimethylammoniumbromid, Polyoxyethylenstearaten, kolloidalem Siliciumdioxid, Phosphaten, Natriumdodecylsulfat, Carboxymethylcellulose-Kalzium, Hydroxypropylcellulosen, Hypromellose, Carboxymethylcellulose-Natrium, Methylcellulose, Hydroxyethylcellulose, Hypromellosephthalat, nicht-kristalline Cellulose, Magnesium-Aluminium-Silikat, Triethanolamin, Polyvinylalkohol, Polyvinylpyrrolidon, 4-(1,1,3,3-Tetramethylbutyl)-phenol-Polymer mit Ethylenoxid und Formaldehyd, Poloxameren; Poloxaminen, einem geladenen Phospholipid, Dioctylsulfosuccinat, Dialkylestern der Natriumsulfosuccinylsäure, Natriumlaurylsulfat, Alkylarylpolyethersulfonaten, Gemischen aus Sucrosestearat und Sucrosedistearat, p-Isononylphenoxypoly-(glycidol), Decanoyl-N-methylglucamid; n-Decyl-β-D-glucopyranosid; n-Decyl-β-D-maltopyranosid; n-Dodecyl-β-D-glucopyranosid; n-Dodecyl-β-D-maltosid; Heptanoyl-N-methylglucamid; n-Heptyl-β-D-glucopyranosid; n-Heptyl-β-D-thioglucosid; n-Hexyl β-D-glucopyranosid; Nonanoyl-N-methylglucamid; n-Noyl-β-D-glucopyranosid; Octanoyl-N-methylglucamid; n-Octyl-β-D-glucopyranosid; Octyl-β-D-thioglucopyranosid; Lysozymen, PEG-Phospholipid, PEG-Cholesterin, PEG-Cholesterin-Derivate, PEG-Vitamin A, Random-Copolymeren aus Vinylacetat und Vinylpyrrolidon, kationischen Polymeren, kationischen Biopolymeren, kationischen Polysacchariden, kationischen Cellulosederivaten, kationischen Alginaten, kationischen nichtpolymeren Verbindungen, kationischen Phospholipiden, kationischen Lipiden, Polymethylmethacrylat-TrimethylammoniumBromid, Sulfoniumverbindungen, Polyvinylpyrrolidon-2-Dimethylaminoethyl-Methacrylat-Dimethylsulfat, Hexadecyltrimethylammoniumbromid, Phosphoniumverbindungen, quartären Ammoniumverbindungen, Benzyl-di(2-chloroethyl)ethylammoniumbromid, Kokosnuss Trimethylammoniumchlorid, Kokosnuss Trimethylammoniumbromid, Kokosnuss Methyldihydroxyethylammoniumchlorid, Kokosnuss Methyldihydroxyethylammoniumbromid, Decyltriethylammoniumchlorid, Decyldimethylhydroxyethylammoniumchlorid, Decyldimethylhydroxyethylammoniumchloridbromid, C₁₂₋₁₅Dimethylhydroxyethylammoniumchlorid, C₁₂₋₁₅Dimethylhydroxyethylammoniumchloridbromid, Kokosnuss Dimethylhydroxyethylammoniumchlorid, Kokosnuss Dimethylhydroxyethylammoniumbromid, Myristyltrimethylammoniummethylsulfat, Lauryldimethylbenzylammoniumchlorid, Lauryldimethylbenzylammoniumbromid, Lauryldimethyl(ethenoxy)₄ammoniumchlorid, Lauryldimethyl(ethenoxy)₄ammoniumbromid, N-Alkyl-(C₁₂₋₁₈)-dimethylbenzylammoniumchlorid, N-Alkyl-(C₁₄₋₁₈)-dimethylbenzylammoniumchlorid, N-Tetradecylidmethylbenzylammoniumchlorid-monohydrat, Dimethyldidecylammoniumchlorid, N-Alkyl- und (C₁₂₋₁₄)-dimethyl-1-naphtylmethylammoniumchlorid, Trimethylammoniumhalogenid, Alkyltrimethylammoniumsalzen, Dialkyldimethylammoniumsalzen, Lauryltrimethylammoniumchlorid, ethoxyliertem Alkyamidoalkyldialkylammoniumsalz, einem ethoxyliertem Trialkylammoniumsalz, Dialkylbenzoldialkylammoniumchlorid, N-Didecyldimethylammoniumchlorid, N-Tetradecyldimethylbenzylammoniumchlorid-Monohydrat, N-Alkyl-(C₁₂₋₁₄)-dimethyl-1-naphthylmethylammoniumchlorid, Dodecyldimethylbenzylammoniumchlorid, Dialkylbenzolalkylammoniumchlorid, Laurytrimethylammoniumchlorid, Alkylbenzylmethylammoniumchlorid, Alkylbenzyldimethylammoniumbromid, C₁₂-Trimethylammoniumbromiden, C₁₅-Trimethylammoniumbromiden, C₁₇-Trimethylammoniumbromiden, Dodecylbenzyltriethylammoniumchlorid, Polydiallyldimethylammoniumchlorid (DADMAC), Dimethylammoniumchloriden, Alkyldimethylammoniumhalogeniden, Tricetylmethylammoniumchlorid, Decyltrimethylammoniumbromid, Dodecyltriethylammoniumbromid, Tetradecyltrimethylammoniumbromid, Methyltrioctylammoniumchlorid, POLYQUAT10^{™}, Tetrabutylammoniumbromid, Benzyltrimethylammoniumbromid, Cholineestern, Benzalkoniumchlorid, Stearalkoniumchlorid-Verbindungen, Cetylpyridiniumbromid, Cetylpyridiniumchlorid, Halogenidsalzen von quaternisierten Polyoxyethylalkylaminen, MIRAPOL^{™}, ALKAQUAT^{™}, Alkylpyridiniumsalzen; Aminen, Aminsalzen, Aminoxiden, Imidazoliniumsalzen, protonierten quarternären Acrylamiden, methylierten quarternären Polymeren und kationischem Guar.

7. Zusammensetzung nach Anspruch 6, wobei die Zusammensetzung bioadhäsiv ist.

8. Zusammensetzung nach einem der Ansprüche 1 bis 7, umfassend mindestens einen primären Oberflächenstabilisator und mindestens einen sekundären Oberflächenstabilisator.

9. Zusammensetzung nach einem der Ansprüche 1 bis 8, umfassend als Oberflächenstabilisator Polyvinylpyrrolidon, Natriumdocusat, Lysozym oder eine Kombination davon.

10. Zusammensetzung nach einem der Ansprüche 1 bis 9, ferner umfassend mindestens eine zusätzliche Metaxalon-Zusammensetzung mit einer effektiven durchschnittlichen Partikelgröße, die sich von der effektiven durchschnittlichen Partikelgröße der Zusammensetzung von Anspruch 1 unterscheidet.

11. Zusammensetzung nach einem der Ansprüche 1 bis 10, zusätzlich umfassend eine oder mehrere Wirkstoffe, die nicht Metaxalon sind.

12. Zusammensetzung nach Anspruch 11, wobei:
(a) der oder die zusätzlichen Wirkstoffe, die nicht Metaxalon sind, ausgewählt sind aus der Gruppe bestehend aus Aminosäuren, Proteinen, Peptiden, Nukleotiden, Antiadiposita, Stimulanzien für das zentrale Nervensystem, Carotehoide, Corticosteroiden, Elastaseinhibitoren, Antimykotika, Onkotherapeutika, Antiemetika, Analgetika, kardiovaskulären Mitteln, entzündungshemmenden Mitteln, Anthelmintika, Antiarrhythmika, Antibiotika, Antikoagulationsstoffe, Antidepressiva, antidiabetischen Mitteln, Antiepileptika, Antihistaminika, antihypertensiven Mitteln, antimuskarinischen Mitteln, antimykobakteriellen Mitteln, antineoplastischen Mitteln, Immunsuppressiva, Thyreostatika, antiviralen Mitteln, Anxiolytika, Sedativa, Adstringenzien, Alpha-Adrenorezeptoren-Blockern, Beta-Adrenorezeptoren-Blockern, Blutprodukten, Blutsubstituten, Inotropika, Kontrastmitteln, Corticosteroiden, Hustenunterdrückungsmitteln, diagnostischen Mitteln, diagnostischen bildgebenden Mitteln, Diuretika, Dopaminergika, Hämostatika, immunologischen Mitteln, fettregulierenden Mitteln, muskelrelaxierenden Mitteln, Parasympathomimetika, Parathyroida-Calcitonin, Parathyroida-Biphosphonaten, Prostaglandinen, Radiopharmazeutika, Sexualhormonen, Antiallergika, Stimulanzien, Appetitzügler, Sympathomimetika, Schilddrüsenmitteln, Vasodilatoren, Xanthinen, Aciclovir, Alprazolam, Altretamin, Amilorid, Amiodaron, Benztropinmesylat, Bupropion, Cabergolin, Candesartan, Cerivastatin, Chlorpromazin, Ciprofloxacin, Cisaprid, Clarithromycin, Clonidin, Clopidogrel, Cyclobenzaprin, Cyproheptadin, Delavirdin, Desmopressin, Diltiazem, Dipyridamol, Dolasetron, Enalaprilmaleat, Enalaprilat, Famotidin, Felodipin, Furazolidon, Glipizid, Irbesartan, Ketoconazol, Lansoprazol, Loratadin, Loxapin, Mebendazol, Mercaptopurin, Milrinonlaktat, Minocyclin, Mitoxantron, Nelfinavirmesylat, Nimodipin, Norfloxacin, Olanzapin, Omeprazol, Penciclovir, Pimozid, Tacolimus, Quazepam, Raloxifen, Rifabutin, Rifampin, Risperidon, Rizatriptan, Saquinavir, Sertralin, Sildenafil, Acetylsulfisoxazol, Temazepam, Thiabendazol, Thioguanin, Trandolapril, Triamteren, Trimetrexat, Troglitazon, Trovafloxacin, Verapamil, Vinblastinsulfat, Mycophenolat, Atovaquon, Proguanil, Ceftazidim, Cefuroxim, Etoposid, Terbinafin, Thalidomid, Fluconazol, Amsacrin, Dacarbazin, Teniposid, Acetylsalicylat, einem NSAID und einem COX-2-Inhibitor; und/oder
(b) der oder die zusätzlichen Wirkstoffe, die nicht Metaxalon sind, ein NSAID ist, ausgewählt aus der Gruppe bestehend aus Nabumeton, Tiaramid, Proquazon, Bufexamac, Flumizol, Epirazol, Tinoridin, Timegadin, Dapson, Aspirin, Diflunisal, Benorylat, Fosfosal, Diclofenac, Alclofenac, Fenclofenac, Etodolac, Indomethacin, Sulindac, Tolmetin, Fentiazac, Tilomisol, Carprofen, Fenbufen, Flurbiprofen, Ketoprofen, Oxaprozin, Suprofen, Tiaprofensäure, Ibuprofen, Naproxen, Fenoprofen, Indoprofen, Pirprofen, Flufenaminsäure, Mefenaminsäure, Meclofenaminsäure, Nifluminsäure, Oxyphenbutazon, Phenylbutazon, Apazon, Feprazon, Piroxicam, Sudoxicam, Isoxicam und Tenoxicam; und/oder
(c) der oder die zusätzlichen Wirkstoffe, die nicht Metaxalon sind, ein COX-2-Inhibitor ist, ausgewählt aus der Gruppe bestehend aus Celecoxib, Rofecoxib, Meloxicam, Valdecoxib, Parecoxib, Etoricoxib, SC-236, NS-398, SC-58125, SC-57666, SC-558, SC-560, Etodolac, DFU, Montelukast, L-745337, L-761066, L-761000, L-748780, DUP-697, PGV 20229, Iguratimod, BF 389, PD 136005, PD 142893, PD 145065, PD 138387, Flurbiprofen, Nimesulid, Nabumeton, Flosulid, Piroxicam, Diclofenac, Lumiracoxib, D 1367, Diflumidon, JTE-522, FK-3311, FK 867, FR 115068, GR 253035, RWJ 63556, RWJ 20485, ZK 38997, S 2474, CL 1004, RS 57067, RS 104897, RS 104894, SC 41930, Pranlukast und SB 209670, Heptinylsulfid und FR 140423.

13. Zusammensetzung nach einem der Ansprüche 1 bis 12, wobei:
(a) die Metaxalon-Partikel bei Verabreichung an einen Säuger so redispergieren, dass die Partikel eine effektive durchschnittliche Partikelgröße haben, die ausgewählt ist aus der Gruppe bestehend aus weniger als etwa 2 µm, weniger als etwa 1900 nm, weniger als etwa 1800 nm, weniger als etwa 1700 nm, weniger als etwa 1600 nm, weniger als etwa 1500 nm, weniger als etwa 1400 nm, weniger als etwa 1300 nm, weniger als etwa 1200 nm, weniger als etwa 1100 nm, weniger als etwa 1000 nm, weniger als etwa 900 nm, weniger als etwa 800 nm, weniger als etwa 700 nm, weniger als etwa 600 nm, weniger als etwa 500 nm, weniger als etwa 400 nm, weniger als etwa 300 nm, weniger als etwa 250 nm, weniger als etwa 200 nm, weniger als etwa 150 nm, weniger als etwa 100 nm, weniger als etwa 75 nm und weniger als 50 nm; und/oder
(b) die Zusammensetzung so in einem biorelevanten Medium redispergiert, dass die Metaxalon-Partikel eine effektive durchschnittliche Partikelgröße haben, die ausgewählt ist aus der Gruppe bestehend aus weniger als etwa 2 µm, weniger als etwa 1900 nm, weniger als etwa 1800 nm, weniger als etwa 1700 nm, weniger als etwa 1600 nm, weniger als etwa 1500 nm, weniger als etwa 1400 nm, weniger als etwa 1300 nm, weniger als etwa 1200 nm, weniger als etwa 1100 nm, weniger als etwa 1000 nm, weniger als etwa 900 nm, weniger als etwa 800 nm, weniger als etwa 700 nm, weniger als etwa 600 nm, weniger als etwa 500 nm, weniger als etwa 400 nm, weniger als etwa 300 nm, weniger als etwa 250 nm, weniger als etwa 200 nm, weniger als etwa 150 nm, weniger als etwa 100 nm, weniger als etwa 75 nm und weniger als etwa 50 nm.

14. Zusammensetzung nach Anspruch 13, wobei das biorelevante Medium ausgewählt ist aus der Gruppe bestehend aus Wasser, wässrigen Elektrolytlösungen, wässrigen Salzlösungen, wässrigen Säurelösungen, wässrigen basischen Lösungen und Kombinationen davon.

15. Zusammensetzung nach einem der Ansprüche 1 bis 14, wobei:
(a) die Tₘₐₓ vom Metaxalon bei Untersuchung nach der Verabreichung an einen Säuger in dessen Plasma kleiner als die Tₘₐₓ für eine nicht-nanopartikuläre Metaxalon-Formulierung ist, die in derselben Dosis verabreicht wird; und/oder
(b) die Tₘₐₓ ausgewählt ist aus der Gruppe bestehend aus nicht größer als etwa 90 %, nicht größer als etwa 80 %, nicht größer als etwa 70 %, nicht größer als etwa 60 %, nicht größer als etwa 50 %, nicht größer als etwa 30 %, nicht größer als etwa 25 %, nicht größer als etwa 20 %, nicht größer als etwa 15 %, nicht größer als etwa 10 % und nicht größer als etwa 5 % der Tₘₐₓ, die durch eine nicht-nanopartikuläre Metaxalon-Formulierung erzielt wird, die in derselben Dosis verabreicht wird.

16. Zusammensetzung nach einem der Ansprüche 1 bis 15, wobei:
(a) die Cₘₐₓ von Metaxalon bei Untersuchung nach der Verabreichung an einen Säuger in dessen Plasma größer als die Cₘₐₓ für eine nicht-nanopartikuläre Metaxalon-Formulierung ist, die in derselben Dosis verabreicht wird; und/oder
(b) die Cₘₐₓ ausgewählt ist aus der Gruppe bestehend aus mindestens etwa 50 %, mindestens etwa 100 %, mindestens etwa 200 %, mindestens etwa 300 %, mindestens etwa 400 %, mindestens etwa 500 %, mindestens etwa 600 %, mindestens etwa 700 %, mindestens etwa 800 %, mindestens etwa 900 %, mindestens etwa 1000 %, mindestens etwa 1100 %, mindestens etwa 1200 %, mindestens etwa 1300 %, mindestens etwa 1400 %, mindestens etwa 1500 %, mindestens etwa 1600 %, mindestens etwa 1700 %, mindestens etwa 1800 % oder mindestens etwa 1900 % größer als die Cₘₐₓ, die durch eine nicht-nanopartikuläre Formulierung von Metaxalon erzielt wird, die in derselben Dosis verabreicht wird.

17. Zusammensetzung nach einem der Ansprüche 1 bis 16, wobei:
(a) der AUC von Metaxalon bei Untersuchung nach der Verabreichung an einen Säuger in dessen Plasma größer als der AUC für eine nicht-nanopartikuläre Metaxalon-Formulierung ist, die in derselben Dosis verabreicht wird; und/oder
(b) der AUC ausgewählt ist aus der Gruppe bestehend aus mindestens etwa 25 %, mindestens etwa 50 %, mindestens etwa 75 %, mindestens etwa 100 %, mindestens etwa 125 %, mindestens etwa 150 %, mindestens etwa 175 %, mindestens etwa 200 %, mindestens etwa 225 %, mindestens etwa 250 %, mindestens etwa 275 %, mindestens etwa 300 %, mindestens etwa 350 %, mindestens etwa 400 %, mindestens etwa 450 %, mindestens etwa 500 %, mindestens etwa 550 %, mindestens etwa 600 %, mindestens etwa 650 %, mindestens etwa 700 %, mindestens etwa 750 %, mindestens etwa 800 %, mindestens etwa 850 %, mindestens etwa 900 %, mindestens etwa 950 %, mindestens etwa 1000 %, mindestens etwa 1050 %, mindestens etwa 1100 %, mindestens etwa 1150 % oder mindestens etwa 1200 % größer als der AUC, der durch eine nicht-nanopartikuläre Formulierung von Metaxalon erzielt wird, die in derselben Dosis verabreicht wird.

18. Zusammensetzung nach einem der Ansprüche 1 bis 17, die keine erheblich abweichenden Absorptionsniveaus bei Verabreichung auf nicht nüchternen Magen im Vergleich zu nüchternen Magen erzeugt.

19. Zusammensetzung nach Anspruch 18, wobei der Adsorptionsunterschied der erfindungsgemäßen Metaxalon-Zusammensetzung bei Verabreichung auf nicht nüchternen Magen versus nüchternen Magen ausgewählt ist aus der Gruppe bestehend aus weniger als etwa 100 %, weniger als etwa 90 %, weniger als etwa 80 %, weniger als etwa 70 %, weniger als etwa 60 %, weniger als etwa 50 %, weniger als etwa 40 %, weniger als etwa 30 %, weniger als etwa 25 %, weniger als etwa 20 %, weniger als etwa 15 %, weniger als etwa 10 %, weniger als etwa 5 % und weniger als etwa 3 %.

20. Zusammensetzung nach einem der Ansprüche 1 bis 19, wobei die Verabreichung der Zusammensetzung an einen Menschen in nüchternem Zustand bioäquivalent zu der Verabreichung der Zusammensetzung an einen Probanden in nicht nüchternem Zustand ist.

21. Zusammensetzung nach Anspruch 20, wobei "Bioäquivalenz" vorliegt bei:
(a) einem 90 %-Konfidenzintervall von zwischen 0,80 und 1,25 sowohl für Cₘₐₓ als auch für AUC; oder
(b) einem 90 %-Konfidenzintervall von zwischen 0,80 und 1,25 für AUC und einem 90 %-Konfidenzintervall von zwischen 0,70 und 1,43 für Cₘₐₓ.

22. Zusammensetzung nach einem der Ansprüche 1 bis 21, wobei die Zusammensetzung zusätzlich einen oder mehrere pharmazeutisch akzeptable Arzeimittelträger, Trägerstoffe oder eine Kombination davon aufweist.

23. Verwendung einer Zusammensetzung nach einem der Ansprüche 1 bis 22 für die Herstellung eines Medikaments.

24. Verwendung nach Anspruch 23, wobei das Medikament nützlich ist bei der Behandlung von Indikationen ausgewählt aus der Gruppe bestehend aus Indikationen, bei denen üblicherweise muskuloskelettale Relaxantien verwendet werden, degenerativer Osteoarthritis, Bandscheibenvorfall, Spondylitis, Lamektomie, starken muskuloskelettalen Zerrungen, starken muskuloskelettalen Stauchungen, muskuloskelettalem Trauma, zervikaler Radikulopathie, lumbaler Radikulopathie und einer Kombination davon.

25. Verfahren zur Herstellung einer Metaxalon-Zusammensetzung nach einem der Ansprüche 1 bis 22, umfassend das In-Kontakt-Bringen von Metaxalon-Partikeln oder eines Salzes davon mit mindestens einem Oberflächenstabilisator für eine Zeit und unter Bedingungen, die ausreichen, um eine Metaxalon-Zusammensetzung zu liefern, die eine effektive durchschnittliche Partikelgröße von weniger als etwa 2000 nm aufweist.

26. Verfahren nach Anspruch 25, wobei das In-Kontakt-Bringen Mahlen, Nassmahlen, Homogenisieren oder Präzipitation umfasst.

## Revendications

1. Composition comprenant :
(a) des particules de métaxalone ou d'un sel de celle-ci, dans laquelle les particules de métaxalone présentent une taille moyenne de particules effective inférieure à 2 000 nm ; et
(b) au moins un stabilisant de surface, dans laquelle le stabilisant de surface est adsorbé sur la surface des particules de métaxalone,
à condition que la composition ne soit pas composée de 200 mg de succinate de d-alpha-tocophérol ; de 200 mg de Crémophor RH 40 ; de 100 mg de dibéhénate de glycérol (Compritol 888) ; de 80 mg de distéarate de glycérol (Precivol) ; et de 300 mg de métaxalone.

2. Composition selon la revendication 1, dans laquelle la métaxalone est choisie dans le groupe constitué par une phase cristalline, une phase amorphe, une phase semi-cristalline, une phase semi-amorphe, et les mélanges de celles-ci.

3. Composition selon la revendication 1 ou la revendication 2, dans laquelle la taille moyenne de particules effective des particules de métaxalone est choisie dans la gamme constituée par des valeurs inférieure à 1 900 nm, inférieure à 1 800 nm, inférieure à 1 700 nm, inférieure à 1 600 nm, inférieure à 1 500 nm, inférieure à 1 400 nm, inférieure à 1 300 nm, inférieure à 1 200 nm, inférieure à 1 100 nm, inférieure à 1 000 nm, inférieure à 900 nm, inférieure à 800 nm, inférieure à 700 nm, inférieure à 600 nm, inférieure à 500 nm, inférieure à 400 nm, inférieure à 300 nm, inférieure à 200 nm, inférieure à 100 nm, inférieure à 75 nm, et inférieure à 50 nm.

4. Composition selon l'une quelconque des revendications 1 à 3, dans laquelle la composition est formulée :
(a) pour une administration choisie dans le groupe constitué par une administration orale, pulmonaire, rectale, ophtalmique, colique, parentérale, intracistémale, intra-vaginale, intrapéritonéale, locale, buccale, nasale, et topique ; et/ou
(b) en une forme pharmaceutique choisie dans le groupe constitué par des dispersions liquides, des suspensions orales, des gels, des aérosols, des pommades, des crèmes, des formulations à libération modifiée, des formulations à fusion rapide, des formulations lyophilisées, des comprimés, des gélules, des formulations à libération retardée, des formulations à libération prolongée, des formulations à libération pulsatile, et des formulations mixtes à libération immédiate et à libération programmée.

5. Composition selon l'une quelconque des revendications 1 à 4, dans laquelle :
(a) la métaxalone ou un de ses sels est présent en une quantité choisie dans le groupe des valeurs de 99,5% à 0,001%, de 95% à 0,1%, et de 90% à 0,5%, en poids, par rapport au poids combiné total de la métaxalone ou d'un de ses sels et d'au moins un stabilisant de surface, à l'exclusion d'autres excipients ; et
(b) le au moins un stabilisant de surface est présent en une quantité choisie dans le groupe des valeurs de 0,5% à 99,999% en poids, de 5,0% à 99,9% en poids, et de 10% à 99,5% en poids, par rapport au poids sec combiné total de la métaxalone ou d'un de ses sels et d'au moins un stabilisant de surface, à l'exclusion d'autres excipients.

6. Composition selon l'une quelconque des revendications 1 à 5, dans laquelle au moins un stabilisant de surface :
(a) est choisi dans le groupe constitué par un stabilisant de surface non ionique, un stabilisant de surface anionique, un stabilisant de surface cationique, un stabilisant de surface zwitterionique, et un stabilisant de surface ionique ; et/ou
(b) est choisi dans le groupe constitué par le chlorure de cétylpyridinium, la gélatine, la caséine, les phosphatides, le dextrane, le glycérol, la gomme arabique, le cholestérol, la gomme adragante, l'acide stéarique, le chlorure de benzalkonium, le stéarate de calcium, le monostéarate de glycérol, l'alcool cétostéarylique, la cire émulsifiante cétomacrogol, les esters de sorbitan, les éthers polyoxyéthylène d'alkyle, les dérivés d'huile de ricin polyoxyéthyléné, les esters d'acides gras et de sorbitan polyoxyéthyléné, les polyéthylèneglycols, le bromure de dodécyltriméthylammonium, les stéarates de polyoxyéthylène, le dioxyde de silicium colloïdal, les phosphates, le dodécylsulfate de sodium, la carboxyméthylcellulose calcique, les hydroxypropylcelluloses, l'hypromellose, la carboxyméthylcellulose sodique, la méthylcellulose, l'hydroxyéthylcellulose, le phtalate d'hypromellose, la cellulose non cristalline, le silicate de magnésium et d'aluminium, la triéthanolamine, l'alcool polyvinylique, la polyvinylpyrrolidone, un polymère de 4-(1,1,3,3-tétraméthylbutyl)-phénol avec l'oxyde d'éthylène et le formaldéhyde, les poloxamères ; les poloxamines, un phospholipide chargé, le dioctylsulfosuccinate, les esters dialkyliques de sulfosuccinate sodique, le laurylsulfate de sodium, les alkylarylpolyéthersulfonates, les mélanges de stéarate de saccharose et de distéarate de saccharose, le p-isononylphénoxypoly-(glycidol), le décanoyl-N-méthylglucamide ; le n-décyl-β-D-glucopyranoside ; le n-décyl-β-D-maltopyranoside ; le n-dodécyl-β-D-glucopyranoside ; le n-dodécyl-β-D-maltoside ; l'heptanoyl-N-méthylglucamide ; le n-heptyl-β-D-glucopyranoside ; le n-heptyl-β-D-thioglucoside ; le n-hexyl-β-D-glucopyranoside ; le nonanoyl-N-méthylglucamide ; le n-nonyl-β-D-glucopyranoside ; l'octanoyl-N-méthylglucamide ; le n-octyl-β-D-glucopyranoside ; l'octyl-β-D-thioglucopyranoside ; le lysozyme, le PEG-phospholipide, le PEG-cholestérol, un dérivé de PEG-cholestérol, le PEG-vitamine A, les copolymères statistiques d'acétate de vinyle et de vinylpyrrolidone, les polymères cationiques, les biopolymères cationiques, les polysaccharides cationiques, les celluloses cationiques, les alginates cationiques, les composés non polymères cationiques, les phospholipides cationiques, les lipides cationiques, le poly(méthacrylate de méthyle), le bromure de triméthylammonium, les composés sulfonium, le diméthylsulfate de méthacrylate de polyvinylpyrrolidone-2-diméthylaminoéthyle, le bromure d'hexadécyltriméthylammonium, les composés phosphonium, les composés ammonium quaternaire, le bromure de benzyl-di(2-chloroéthyl)éthylammonium, le chlorure de coprah-triméthylammonium, le bromure de coprah-triméthylammonium, le chlorure de coprahméthyldihydroxyéthylammonium, le bromure de coprahméthyldihydroxyéthylammonium, le chlorure de décyltriéthylammonium, le chlorure de décyldiméthylhydroxyéthylammonium, le chlorure et bromure de décyldiméthylhydroxyéthylammonium, le chlorure d'alkyl(en C₁₂ à C₁₅)-diméthylhydroxyéthylammonium, le bromure d'alkyl(en C₁₂ à C₁₅)-diméthylhydroxyéthylammonium, le chlorure de coprah-diméthyl-hydroxyéthylammonium, le bromure de coprah-diméthyl-hydroxyéthylammonium, le méthylsulfate de myristyltriméthylammonium, le chlorure de lauryldiméthyl-benzylammonium, le bromure de lauryldiméthyl-benzylammonium, le chlorure de lauryldiméthyl-(éthénoxy)₄-ammonium, le bromure de lauryldiméthyl-(éthénoxy)₄-ammonium, le chlorure de N-alkyl(en C₁₂ à C₁₈)-diméthylbenzylammonium, le chlorure de N-alkyl(en C₁₄ à C₁₈)-diméthylbenzylammonium, le chlorure de N-tétradécylidméthylbenzylammonium monohydraté, le chlorure de diméthyldidécylammonium, le chlorure de N-alkyl(en C₁₂ à C₁₄)-diméthyl-1-napthylméthylammonium, l'halogénure de triméthylammonium, les sels d'alkyltriméthylammonium, les sels de dialkyldiméthylammonium, le chlorure de lauryltriméthylammonium, le sel d'alkylamidoalkyldialkylammonium éthoxylé, un sel de trialkylammonium éthoxylé, le chlorure de dialkylbenzène-dialkylammonium, le chlorure de N-didécyldiméthylammonium, le chlorure de N-tétradécyldiméthylbenzylammonium monohydraté, le chlorure de N-alkyl(en C₁₂ à C₁₄)-diméthyl-1-naphtylméthylammonium, le chlorure de dodécyldiméthylbenzylammonium, le chlorure de dialkylbenzènealkylammonium, le chlorure de lauryltriméthylammonium, le chlorure d'alkylbenzylméthylammonium, le bromure d'alkylbenzyldiméthylammonium, les bromures d'alkyl(en C₁₂)-triméthylammonium, les bromures d'alkyl(en C₁₅)-triméthylammonium, les bromures d'alkyl(en C₁₇)-triméthylammonium, le chlorure de dodécylbenzyltriéthylammonium, le poly(chlorure de diallyldiméthylammonium) (DADMAC), les chlorures de diméthylammonium, les halogénures d'alkyldiméthylammonium, le chlorure de tricétylméthylammonium, le bromure de décyltriméthylammonium, le bromure de dodécyltriéthylammonium, le bromure de tétradécyltriméthylammonium, le chlorure de méthyltrioctylammonium, POLYQUAT 10^{™}, le bromure de tétrabutylammonium, le bromure de benzyltriméthylammonium, les esters de choline, le chlorure de benzalkonium, les composés du chlorure de stéaralkonium, le bromure de cétylpyridinium, le chlorure de cétylpyridinium, les halogénures de polyoxyéthylalkylamines quaternisées, MIRAPOL^{™}, ALKAQUAT^{™}, les sels d'alkylpyridinium ; les amines, les sels d'amines, les oxydes d'amines, les sels d'imidazolinium, les acrylamides quaternaires protonés, les polymères quaternaires méthylés, et la gomme guar cationique.

7. Composition selon la revendication 6, dans laquelle la composition est un bio-adhésif.

8. Composition selon l'une quelconque des revendications 1 à 7, comprenant au moins un stabilisant de surface primaire et au moins un stabilisant de surface secondaire.

9. Composition selon l'une quelconque des revendications 1 à 8, comprenant, en tant que stabilisant de surface, la polyvinylpyrrolidone, le docusate de sodium, le lysozyme, ou une combinaison de ceux-ci.

10. Composition selon l'une quelconque des revendications 1 à 9, comprenant en outre au moins une composition de métaxalone supplémentaire ayant une taille moyenne de particules effective qui est différente de la taille moyenne de particules effective de la composition de métaxalone selon la revendication 1.

11. Composition selon l'une quelconque des revendications 1 à 10, comprenant en outre un ou plusieurs principes actifs non-métaxalone.

12. Composition selon la revendication 11, dans laquelle :
(a) ledit un ou plusieurs principe actif non-métaxalone supplémentaire est choisi dans le groupe constitué par les acides aminés, les protéines, les peptides, les nucléotides, les médicaments destinés au traitement de l'obésité, les stimulants du système nerveux central, les caroténoïdes, les corticostéroïdes, les inhibiteurs de l'élastase, les antifongiques, les agents de thérapie oncologique, les anti-émétiques, les analgésiques, les agents cardio-vasculaires, les agents anti-inflammatoires, les vermifuges, les agents anti-arythmiques, les antibiotiques, les anticoagulants, les antidépresseurs, les agents antidiabétiques, les anti-épileptiques, les antihistaminiques, les agents antihypertenseurs, les agents antimuscariniques, les agents antimycobactériens, les agents antinéoplasiques, les immunosuppresseurs, les agents antithyroïdiens, les agents antiviraux, les anxiolytiques, les sédatifs, les astringents, les agents de blocage du récepteur alpha-adrénergique, les agents de blocage du bêta-adrénocepteur, les produits sanguins, les substituts sanguins, les agents inotropes cardiaques, les milieux de contraste, les corticostéroïdes, les antitussifs, les agents diagnostiques, les agents d'imagerie diagnostique, les diurétiques, les dopaminergiques, les hémostatiques, les agents immunologiques, les agents de régulation du métabolisme des lipides, les myorelaxants, les agents parasympathomimétiques, la calcitonine parathyroïde, les biphosphonates parathyroïdes, les prostaglandines, les produits pharmaceutiques radiologiques, les hormones sexuelles, les agents anti-allergiques, les stimulants, les anorexigènes, les sympathomimétiques, les agents thyroïdiens, les vasodilatateurs, les xanthines, l'acyclovir, l'alprazolam, l'altrétamine, l'amiloride, l'amiodarone, le mésylate de benztropine, le bupropion, la cabergoline, le candesartan, la cérivastatine, la chlorpromazine, la ciprofloxacine, le cisapride, la clarithromycine, la clonidine, le clopidogrel, la cyclobenzaprine, la cyproheptadine, la délavirdine, la desmopressine, le diltiazem, le dipyridamole, le dolasétron, le maléate d'énalapril, l'énalaprilate, la famotidine, la félodipine, la furazolidone, le glipizide, l'irbesartan, le kétoconazole, le lansoprazole, la loratadine, la loxapine, le mébendazole, la mercaptopurine, le lactate de milrinone, la minocycline, la mitoxantrone, le mésylate de nelfinavir, la nimodipine, la norfloxacine, l'olanzapine, l'oméprazole, le penciclovir, le pimozide, le tacolimus, le quazepam, le raloxifenc, la rifabutine, la rifampine, la risperidone, le rizatriptan, le saquinavir, la sertraline, le sildénafil, l'acétyl-sulfisoxazole, le témazépam, le thiabendazole, la thioguanine, le trandolapril, le triamtérène, le trimétrexate, la troglitazone, la trovafloxacine, le vérapamil, le sulfate de vinblastine, le mycophénolate, l'atovaquone, l'atovaquone, le proguanil, la ceftazidime, la céfuroxime, l'étoposide, la terbinafine, le thalidomide, le fluconazole, l'amsacrine, la dacarbazine, le téniposide, l'acétylsalicylate, un AINS, et un inhibiteur de COX-2 ; et/ou
(b) ledit un ou plusieurs principe actif non-métaxalone supplémentaire est un AINS choisi dans le groupe constitué par la nabumétone, le tiaramide, la proquazone, le buféxamac, le flumizole, l'épirazole, la tinoridine, la timégadine, la dapsone, l'aspirine, le diflunisal, le benorylate, le fosfosal, le diclofénac, l'alclofénac, le fénclofénac, l'étodolac, l'indométhacine, le sulindac, la tolmétine, le fentiazac, le tilomisole, le carprofène, le fenbufène, le flurbiprofène, le kétoprofène, l'oxaprozine, le suprofène, l'acide tiaprofénique, l'ibuprofène, le naproxène, le fénoprofène, l'indoprofène, le pirprofène, l'acide flufénamique, l'acide méfénamiqie, l'acide méclofenamique, l'acide niflumique, l'oxyphénbutazone, la phénylbutazone, l'apazone, le féprazone, le piroxicam, le sudoxicam, l'isoxicam, et le ténoxicam ; et/ou
(c) ledit un ou plusieurs principe actif non-métaxalone supplémentaire est un inhibiteur de COX-2 choisi dans le groupe constitué par le célécoxib, le rofécoxib, le méloxicam, le valdécoxib, le parécoxib, l'étoricoxib, SC-236, NS-398, SC-58125, SC-57666, SC-558, SC-560, l'étodolac, DFU, le monteleukast, L-745337, L-761066, L-761000, L-748780, DUP-697, PGV 20229, l'iguratimod, BF 389, PD 136005, PD 142893, PD 145065, PD 138387, le flurbiprofène, le nimésulide, le nabumétone, le flosulide, le piroxicam, le diclofénac, le lumiracoxib, D 1367, la diflumidone, JTE-522, FK-3311, FK 867, FR 115068, GR 253035, RWJ 63556, RWJ 20485, ZK 38997, S 2474, CL 1004, RS 57067, RS 104897 RS 104894, SC 41930, le pranlukast, et SB 209670, l'heptinylsulfure, et FR 140423.

13. Composition selon l'une quelconque des revendications 1 à 12, dans laquelle :
(a) lors de l'administration à un mammifère, les particules de métaxalone sont redispersées si bien que les particules ont une taille moyenne de particules effective choisie dans le groupe des valeurs inférieure à environ 2 micromètres, inférieure à environ 1 900 nm, inférieure à environ 1 800 nm, inférieure à environ 1 700 nm, inférieure à environ 1 600 nm, inférieure à environ 1 500 nm, inférieure à environ 1 400 nm, inférieure à environ 1 300 nm, inférieure à environ 1 200 nm, inférieure à environ 1 100 nm, inférieure à environ 1 000 nm, inférieure à environ 900 nm, inférieure à environ 800 nm, inférieure à environ 700 nm, inférieure à environ 600 nm, inférieure à environ 500 nm, inférieure à environ 400 nm, inférieure à environ 300 nm, inférieure à environ 250 nm, inférieure à environ 200 nm, inférieure à environ 150 nm, inférieure à environ 100 nm, inférieure à environ 75 nm, et inférieure à environ 50 nm ; et/ou
(b) la composition est redispersée dans un milieu à visée biologique si bien que les particules ont une taille moyenne de particules effective choisie dans le groupe des valeurs inférieure à 2 micromètres, inférieure à environ 1 900 nm, inférieure à environ 1 800 nm, inférieure à environ 1 700 nm, inférieure à environ 1 600 nm, inférieure à environ 1 500 nm, inférieure à environ 1 400 nm, inférieure à environ 1 300 nm, inférieure à environ 1 200 nm, inférieure à environ 1 100 nm, inférieure à environ 1 000 nm, inférieure à environ 900 nm, inférieure à environ 800 nm, inférieure à environ 700 nm, inférieure à environ 600 nm, inférieure à environ 500 nm, inférieure à environ 400 nm, inférieure à environ 300 nm, inférieure à environ 250 nm, inférieure à environ 200 nm, inférieure à environ 150 nm, inférieure à environ 100 nm, inférieure à environ 75 nm, et inférieure à environ 50 nm.

14. Composition selon la revendication 13, dans laquelle le milieu à visée biologique est choisi dans le groupe constitué par l'eau, les solutions électrolytiques aqueuses, les solutions aqueuses d'un sel, les solutions aqueuses d'un acide, les solutions aqueuses d'une base, et leurs combinaisons.

15. Composition selon l'une quelconque des revendications 1 à 14, dans laquelle :
(a) la valeur Tₘₐₓ de la métaxalone, lorsque celle-ci est dosée dans le plasma d'un sujet mammifère à la suite d'une administration, est inférieure à la valeur Tₘₐₓ d'une formulation de métaxalone non-nanoparticulaire, administrée avec le même dosage ; et/ou
(b) la valeur Tₘₐₓ est choisie dans le groupe constitué par les valeurs non supérieure à environ 90%, non supérieure à environ 80%, non supérieure à environ 70%, non supérieure à environ 60%, non supérieure à environ 50%, non supérieure à environ 30%, non supérieure à environ 25%, non supérieure à environ 20%, non supérieure à environ 15%, non supérieure à environ 10%, et non supérieure à environ 5% de la valeur Tₘₐₓ présentée par une formulation de métaxalone non-nanoparticulaire, administrée avec le même dosage.

16. Composition selon l'une quelconque des revendications 1 à 15, dans laquelle :
(a) la valeur Cₘₐₓ de la métaxalone, lorsque celle-ci est dosée dans le plasma d'un sujet mammifère à la suite d'une administration, est supérieure à la valeur Cₘₐₓ d'une formulation de métaxalone non-nanoparticulaire, administrée avec le même dosage ; et/ou
(b) la valeur Cₘₐₓ est choisie dans le groupe constitué par les valeurs d'au moins environ 50%, d'au moins environ 100%, d'au moins environ 200%, d'au moins environ 300%, d'au moins environ 400%, d'au moins environ 500%, d'au moins environ 600%, d'au moins environ 700%, d'au moins environ 800%, d'au moins environ 900%, d'au moins environ 1000%, d'au moins environ 1100%, d'au moins environ 1200%, d'au moins environ 1300%, d'au moins environ 1400%, d'au moins environ 1500%, d'au moins environ 1600%, d'au moins environ 1700%, d'au moins environ 1800%, ou d'au moins environ 1900% supérieure à la valeur Cₘₐₓ présentée par une formulation de métaxalone non-nanoparticulaire, administrée avec le même dosage.

17. Composition selon l'une quelconque des revendications 1 à 16, dans laquelle :
(a) l'aire sous la courbe de la métaxalone, lorsque celle-ci est dosée dans le plasma d'un sujet mammifère suivant l'administration, est supérieure à l'aire sous la courbe d'une formulation de métaxalone non-nanoparticulaire, administrée avec le même dosage ; et/ou
(b) l'aire sous la courbe est choisie dans le groupe constitué par les valeurs d'au moins environ 25%, d'au moins environ 50%, d'au moins environ 75%, d'au moins environ 100%, d'au moins environ 125%, d'au moins environ 150%, d'au moins environ 175%, d'au moins environ 200%, d'au moins environ 225%, d'au moins environ 250%, d'au moins environ 275%, d'au moins environ 300%, d'au moins environ 350%, d'au moins environ 400%, d'au moins environ 450%, d'au moins environ 500%, d'au moins environ 550%, d'au moins environ 600%, d'au moins environ 750%, d'au moins environ 700%, d'au moins environ 750%, d'au moins environ 800%, d'au moins environ 850%, d'au moins environ 900%, d'au moins environ 950%, d'au moins environ 1000%, d'au moins environ 1050%, d'au moins environ 1100%, d'au moins environ 1150%, ou d'au moins environ 1200% supérieure à l'aire sous la courbe présentée par la formulation de métaxalone non-nanoparticulaire, administrée avec le même dosage.

18. Composition selon l'une quelconque des revendications 1 à 17 qui ne produit pas de niveaux d'absorption significativement différents lorsqu'elle est administrée dans des conditions d'alimentation par rapport à des conditions de jeun.

19. Composition selon la revendication 18, dans laquelle la différence d'absorption de la composition de métaxalone de la présente invention, lorsque celle-ci est administrée à l'état alimenté par rapport à l'état à jeun, est choisie dans le groupe constitué par une valeur inférieure à environ 100%, inférieure à environ 90%, inférieure à environ 80%, inférieure à environ 70%, inférieure à environ 60%, inférieure à environ 50%, inférieure à environ 40%, inférieure à environ 30%, inférieure à environ 25%, inférieure à environ 20%, inférieure à environ 15%, inférieure à environ 10%, inférieure à environ 5%, et inférieure à environ 3%.

20. Composition selon l'une quelconque des revendications 1 à 19, dans laquelle, sur le plan biologique, l'administration de la composition à un être humain dans un état à jeun est équivalente à une administration de la composition à un sujet à l'état alimenté.

21. Composition selon la revendication 20, dans laquelle "l'équivalence sur le plan biologique" est confirmée par :
(a) un intervalle de confiance à 90% compris entre 0,80 et 1,25 à la fois pour la valeur Cₘₐₓ et l'aire sous la courbe, ou
(b) un intervalle de confiance à 90% compris entre 0,80 et 1,25 pour l'aire sous la courbe et un intervalle de confiance à 90% compris entre 0,70 et 1,43 pour la valeur Cₘₐₓ.

22. Composition selon l'une quelconque des revendications 1 à 21, dans laquelle la composition comprend en outre un ou plusieurs excipients, charges acceptables sur le plan pharmaceutique, ou une combinaison de ceux-ci.

23. Utilisation d'une composition selon l'une quelconque des revendications 1 à 22 pour la préparation d'un médicament.

24. Utilisation selon la revendication 23, dans laquelle le médicament est utilisé pour des indications thérapeutiques choisies dans le groupe constitué par les indications dans lesquelles on utilise habituellement des relaxants musculosqueléttiques telles que l'ostéoarthrite dégénérative, l'hernie discale, la spondylite, la laminectomie, les élongations musculosquelettiques graves, les entorses musculosquelettiques graves, les traumatismes musculosquelettiques, la radiculopathie cervicale, la radiculopathie lombaire, et une combinaison de celles-ci.

25. Procédé de fabrication d'une composition de métaxalone selon l'une quelconque des revendications 1 à 22, comprenant la mise en contact de particules de métaxalone ou d'un sel de celle-ci avec au moins un stabilisant de surface pendant une durée et dans des conditions suffisantes pour fournir une composition de métaxalone ayant une taille moyenne de particules effective inférieure à environ 2 000 nm.

26. Procédé selon la revendication 25, dans lequel ladite mise en contact comprend un broyage, un broyage par voie humide, une homogénéisation, ou une précipitation.
